# EUROPEAN PATENT APPLICATION

(11) **EP 2 386 854 A1**
(43) Date of publication of application: **16.11.2011**
(21) Application number: 11156786.3
(22) Date of filing: 18.04.2006
(51) Int. Cl.: G01N 33/48, G01N 31/00, G01N 33/574

(54) **Diagnostic markers of breast cancer treatment and progression and methods of use thereof**

(30) Priority: 19.04.2005 US 673223 P
(62) Divisional of application: 06750614.7
(71) Applicant: Prediction Sciences LLC, La Jolla CA 92037 (US)
(72) Inventor: Linke, Steven P., La Jolla, CA 92037 (US); Bremer, Troy, Dana Point, CA 92629 (US); Diamond, Cornelius, Leucadia, CA 92024 (US)
(74) Representative: Müller, Christian Stefan Gerd

(57) **Abstract**

To maximize both the life expectancy and quality of life of patients with operable breast cancer, it is important to predict adjuvant treatment outcome and likelihood of progression before treatment. A machine-learning based method is used to develop a cross-validated model to predict (1) the outcome of adjuvant treatment, particularly endocrine treatment outcome, and (2) likelihood of cancer progression before treatment. The model includes standard clinicopathological features (Figure 1), as well as molecular markers collected using standard immunohistochemistry and fluorescence in situ hybridization (Figure 3). The model significantly outperforms the St. Gallen Consensus guidelines and the Nottingham Prognostic Index (Figure 11), thus providing a clinically useful and cost-effective prognostic for breast cancer patients.
We describe a method of predicting response to endocrine therapy or predicting disease progression in breast cancer characterized in that
a breast cancer test sample is obtained from a subject;
clinicopathological data is obtained from said breast test sample;
the obtained breast cancer test sample is analyzed for presence or amount of (1) one or more molecular markers of hormone receptor status, one or more growth factor receptor maskers, and one or more tumor suppression/apoptosis molecular markers; (2) one or more additional molecular markers both proteomic and non-proteomic that are indicative of breats cancer disease processes from a group consisting essentially of angiogenesis, apoptosis, catenin/cadherin proliferation/differentiation, cell cycle processes, cell surface processes, cell-cell interaction, cell migration, centrosomal processes, cellular adhesion, cellular proliferation, cellular metastasis, invasion, cytoskeletal processes, ERBB2 interactions, estrogen co-receptors, growth factors and receptors, membrane/integrin/signal transduction, metastasis, oncogenes, proliferation, proliferation oncogenes, signal transduction, surface antigens and transcription factor molecular markers; and then
(1) a presence or amount of said molecular markers is correlated with (2) clinicopathological data from said tissue sample other than the molecular markers of breast cancer disease processes, in order to deduce a probability of response to endocrine therapy of future risk of disease progression in breast cancer for the subject.

## Description

### REFERENCE TO RELATED PATENT APPLICATIONS

The present application is descended from U.S. provisional patent application number 60/673,223, filed April 19, 2005, which is hereby incorporated by reference in its entirety.

### GOVERNMENT SUPPORT

The present invention was developed under Research Support of the United States National Institute of Standards and Technology, Advanced Technology Program, Award #20024937. The U.S. Government may have certain rights in this invention.

### FIELD OF THE INVENTION

The present invention generally pertains to the prediction of the outcome of endocrine, and particularly tamoxifen, treatment of breast cancer based on the presence and quantities of certain protein molecular markers, called biomarkers, present in the treated patients. The present invention also pertains to the prediction of progression of breast cancer, e.g. whether or not the patient's tumour is likely to metastasize, based upon cancer based on the presence and quantities of certain protein molecular markers.

The present invention specifically concerns (1) the identification of groups, or "palettes", of biomarkers particularly useful in combination for enhanced predictive accuracy of patient response to breast cancer therapy with tamoxifen, (2) the identification of certain pairs of biomarkers that, in pairwise combination, are or superior predictive accuracy in the particular estimation of percentage disease-specific survival at most usually and particularly, some 30+ months from onset of tamoxifen treatment, and, commensurate with the predictive accuracy of these biomarker pairs, (3) the recognition and quantification of the significance of any changes in any patient biomarkers, and particularly in those biomarkers that, taken pairwise, are of superior predictive accuracy in advanced stages of breast cancer.

### BACKGROUND OF THE INVENTION

The following discussion of the background of the invention is merely provided to aid the reader in understanding the invention and is not admitted to describe or constitute prior art to the present invention.

Breast cancer is the most common malignancy in Western women, and it is second only to lung cancer as the most common cause of cancer death (See for instance Cancer Facts and Figures 2004. Atlanta, GA, American Cancer Society, 2004). It affects millions of women worldwide (See for instance GLOBOCAN 2002. 2002, http://www-dep.iarc.fr/). The therapeutic options for the treatment of breast cancers are complex and varied, including surgery, radiotherapy, endocrine therapy, and cytotoxic chemotherapy (See for instance Breast cancer (PDQ): Treatment. National Cancer Institute, 2004, http://www.cancer.gov/cancertopics/pdq/treatment/breast/healthprofessional; Breast cancer, National Comprehensive Cancer Network, 2004).

Roughly 75% of breast cancers are positive for the hormone-based estrogen receptor (ER) and/or progesterone receptor (PGR) (See for instance Osborne CK: Steroid hormone receptors in breast cancer management. Breast Cancer Res Treat 51:227-238, 1998). Most of these patients are treated with an endocrine therapy, either as an adjuvant to surgery in early stage disease or as the primary treatment in more advanced disease. The most common endocrine therapy has been the selective estrogen receptor modulator (SERM) tamoxifen (Nolvadex). It has been in use for over 20 years and demonstrably prolongs survival (See for instance Tamoxifen for early breast cancer: an overview of the randomised trials. Early Breast Cancer Trialists' Collaborative Group. Lancet 351:1451-1467,1998).

Binding of estrogen to ER causes its phosphorylation and dimerization, followed by movement into the nucleus and transcription of a variety of genes, including secreted growth and angiogenic factors (See for instance Osborne CK, Shou J, Massarweh S, et al: Crosstalk between estrogen receptor and growth factor receptor pathways as a cause for endocrine therapy resistance in breast cancer. Clin Cancer Res 11:865s-870s, 2005 (suppl)), in a process called nuclear-initiated steroid signalling. There is also evidence of a membrane-bound fraction of ER that can activate other growth pathways, including the EGFR (ERBB1) and ERBB2 pathways (See for instance Shou J, Massarweh S, Osborne CK, et al: Mechanisms of tamoxifen resistance: increased estrogen receptor-HER2/neu cross-talk in ER/HER2-positive breast cancer. J Natl Cancer Inst 96:926- 935, 2004), in a process called membrane-initiated steroid signalling. In breast tissue, tamoxifen competes with estrogen for binding to ER, thereby reducing proliferation through inhibition of ER's nuclear function. However, it also has been reported that tamoxifen can produce a weak agonist effect by stimulating the membrane-initiated signalling pathway when the relevant growth factors (e.g., EGFR and/or ERBB2) are overexpressed and/or by stimulating the nuclear- initiated pathway in the presence of overexpressed coactivators (e.g., NCOA1 and/or NCOA3) (See for instance Smith CL, Nawaz Z, O'Malley BW: Coactivator and corepressor regulation of the agonist/antagonist activity of the mixed antiestrogen, 4-hydroxytamoxifen. Mol Endocrinol 11:657-666, 1997; Osborne CK, Bardou V, Hopp TA, et al: Role of the estrogen receptor coactivator AlB1 (SRC-3) and HER-2/neu in tamoxifen resistance in breast cancer. J Natl Cancer Inst 95:353-361, 2003). Additional mechanisms of cross-talk between the growth factor receptor pathways may also lead to tamoxifen resistance (See for instance Clarke R, Liu MC, Bouker KB, et al: Antiestrogen resistance in breast cancer and the role of estrogen receptor signalling. Oncogene 22:7316-7339, 2003). In fact, approximately 40% of hormone receptor-positive patients fail to respond to tamoxifen (See for instance Nicholson RI, Gee JM, Knowlden J, et al: The biology of antihormone failure in breast cancer. Breast Cancer Res Treat 80 Suppl 1:S29-34; discussion S35, 2003 (suppl); Clarke R, Liu MC, Bouker KB, et al., Ibid*.*)*.*

Related to these agonistic effects, tamoxifen can have a growth stimulatory effect on tissues such as the endometrium, leading to increased risk of endometrial hyperplasia and cancer. Other side effects include deep venous thrombosis and pulmonary emboli, development of benign ovarian cysts, vaginal discharge or irritation and hot flashes, and vision problems. There is also evidence of increased risk of gastrointestinal cancer and stroke (See for instance Breast cancer (PDQ): *Ibid.*)*.*

Recent studies in post-menopausal women have demonstrated the effectiveness of a different class of endocrine therapy drugs, aromatase inhibitors. In contrast to tamoxifen, which competes with estrogen for binding to ER, aromatase inhibitors directly reduce circulating estrogen levels. Thus, patients who might be resistant to tamoxifen due to its agonist characteristics arising from cross-talk with other growth pathways or deregulation of ER coregulators might be sensitive to aromatase inhibitors. Aromatase inhibitors provide longer recurrence-free survival and generally lower risk of endometrial cancer and thromboembolic events. However, improvements in overall survival are not yet clear, and treatments are accompanied by a different set of side effects, including bone fracture risk and arthralgia. Additionally, the long-term consequences of their use are currently unknown, and the treatments are currently quite costly and only recommended in postmenopausal women. Thus, tamoxifen will remain important in adjuvant breast cancer therapy. Accurate treatment outcome prediction could guide patients to the most biologically and cost effective treatments in a timely fashion.

Intense research has been conducted in recent years on molecular markers that could provide prognostic information and/or predict treatment outcome. It will be seen that the study supportive of the present invention served to analyze data on the standard hormone receptors (ER and PGR), as well as the growth factor receptors EGFR and ERBB2. In addition, the tumour suppressors CDKN1B and TP-53, the anti-apoptotic factor BCL2, the proliferation markers CCND1 and KI-67, and the MYC oncogene were among those studied.

Although a number of studies have been published indicating that these markers have or likely have prognostic significance, some studies have not confirmed the findings, and no consensus has been reached on their utility. More importantly; however, the present invention will be seen to demonstrate the importance of the conditional interpretation of certain markers on others due to their interdependency. Some of these studies will be detailed in later sections of the instant invention.

Other research specific to tamoxifen resistance include U.S. patent application 10/418,027 and U.S. patent application 10/177,296 concerning the association of expression levels of the individual protein markers AIB-1 and p38 MAPK, respectively, to tamoxifen response and/or resistance. However, these individual markers by themselves do not have the required sensitivity and/or specificity to be used in the clinic. U.S. patent application 11/061,067 details several multi-marker panels that define patient outcome based upon "...assessing the patient's likely prognosis based upon binding of the panel to the tumor sample." This method is equivalent with a 'voting scheme' in which just the presence or absence of the binding of an antibody is enough to give a prognostic indication. However, as the instant invention describes below and in Figure 11, the scheme detailed in U.S. patent application 11/061,067 is not enough to produce a diagnostic of sufficient sensitivity and specificity. Still other relevant literature to the instant invention include U.S. patent applications 10/872063, 10/883303, and 10/852797, which claim gene-expression tests for predicting breast cancer progression and treatment to various chemotherapies. As described below, gene expression tests have numerous problems in that the relevant genes that are claimed come from examining a number of samples x which is orders of magnitude less than the total number of genes y initially examined. In doing such, it is unlikely from a statistical viewpoint that such gene sets will produce the same sensitivity and specificity as the initial result detailed in U.S. patent applications 10/872063, 10/883303, and 10/852797, and other literature described elsewhere in the initial invention. U.S. patent applications 10/872063, 10/883303, and 10/852797 make mention of the protein products of such genes in producing such a test, but (1) this is not enabled in these patents; (2) the instant invention enables in its claims a minimal set of specific protein product biomarkers interpolated by a specific nonlinear algorithms which allows a highly sensitive and specific test validated by independent testing patient populations, and (3) none of these applications detail usage of clinicopathological variables as part of determining a clinical outcome in conjunction with molecular markers. Beyond the statistical issues, gene expression assays can only measure transcript levels, which do not always correlate with functional protein levels, and they cannot detect protein mislocalization. In addition, the assays are relatively complicated and costly, often requiring sophisticated and/or proprietary technology and multiple steps, including methods to try to reduce the contribution of adjacent non-tumor tissue and to account for RNA degradation.

In contrast, the present invention will be seen to concern the development of a multi-molecular marker diagnostic with significant contributions by ER, PGR, BCL2, ERBB2, KI-67, MYC, TP-53, and others, in addition to standard clinicopathological factors, all interpolated by an algorithm that can deliver superior prognostic ability as compared to individual protein markers or gene expression techniques.

### BRIEF SUMMARY OF THE INVENTION

Provided in the present invention is a method of providing a prognosis of disease-free survival in a cancer patient comprising the steps of obtaining a sample from the patient; and determining various polypeptide levels (e.g. molecular markers) in the sample, wherein change in various polypeptide levels as compared to a control sample indicates the good prognosis of a prolonged disease-free survival. The present invention contemplates a multiple molecular marker diagnostic, the values of each assayed marker collectively interpolated by a non-linear algorithm, to (1) predict the outcomes of endocrine, particularly tamoxifen, therapy for breast cancer in consideration of multiple molecular makers, called biomarkers, of a patient's; and (2) identify whether or not a tumour from a patient is likely to be more aggressive, or malignant, than another and thus requiring neoadjuvant chemotherapy in addition to surgical and radiological treatment. The model was built by multivariate mathematical analysis of (1) many more multiple molecular marker, called biomarkers, than ultimately proved to be significant in combination for forecasting treatment outcomes, in consideration of (2) real-world, clinical, outcomes of real patients who possessed these biomarkers.

The diagnostic is subject to updating, or revision, as any of (1) new biomarkers are considered, (2) new patient data (including as may come from patients who had their own treatment outcomes predicted) becomes available, and/or (3) new (drug) therapies are administered, all without destroying the validity of the instant invention and of discoveries made during the building, and the exercise, thereof, as hereinafter discussed.

A number of different insights are derived from the (1) building the (2) the exercise of the diagnostic. A primary insight may be considered to be the identification of a number, or "palette", of biomarkers that are in combination of superior, and even greatly superior, accuracy for predicting the outcomes of tamoxifen therapy for breast cancer than would be any one, or even two, markers taken alone. This combination's predictive power over that of a simple voting panel response is increased by use of an algorithm that interpolates the linear and non-linear collective contributions of said collection to predict the clinical outcome of interest.

A secondary insight from the diagnostic is that certain biomarkers are or increased predictive accuracy of, in particular, percentage disease-specific survival at 30+ months from onset of treatment when these biomarkers taken in pairs. This does not mean that these biomarker pairs are of overall predictive accuracy to the palette of predictive biomarkers. It only means that, when considered in pairs, certain biomarkers provide useful subordinate predictions.

Finally, a tertiary insight that falls out from the identification of biomarker pairs having superior predictive accuracy is that expected disease-specific survival can, and does, vary greatly when, sometimes, but one single one of these biomarkers changes, as during the course of the treatment of single patient.

Theory of the Invention

In accordance with the present invention, exercise of the diagnostic primarily serves to (1) identify pairs of biomarkers that are unusually strongly related, suggesting in these identified pairs avenues for further investigation of disease pathology, and of drugs; and (2) identify and quantify a palette of biomarkers interpolated by a non-linear algorithm having superior predictive capability for prognosis of outcomes in endocrine therapy of breast.

In another of its aspects, the instant invention is embodied in methods for choosing one or more marker(s) for diagnosis, prognosis, or therapeutic treatment of breast cancer in a patient that together, and as a group, have maximal sensitivity, specificity, and predictive power. Said maximal sensitivity, specificity, and predictive power is in particular realized by choosing one or more markers as constitute a group by a process of plotting receiver operator characteristic (ROC) curves for (1) the sensitivity of a particular combination of markers versus (2) specificity for said combination at various cutoff threshold levels. In addition, the instant invention further discloses methods to interpolate the nonlinear correlative effects of one or more markers chosen by any methodology to such that the interaction between markers of said combination of one or more markers promotes maximal sensitivity, specificity, and predictive accuracy in the diagnosis, prognosis, or therapeutic and treatment of breast cancer.

In various aspects, the present invention relates to (1) materials and procedures for identifying markers that are associated with the diagnosis, prognosis, or differentiation of breast cancer in a patient; (2) using such markers in diagnosing and treating a patient and/or monitoring the course of a treatment regimen; (3) using such markers to identify subjects at risk for one or more adverse outcomes related to breast cancer; and (4) using at one of such markers an outcome marker for screening compounds and pharmaceutical compositions that might provide a benefit in treating or preventing such conditions.

The first three as aspects of the present invention are discussed in the following sections below.

A Palette of Biomarkers Relevant to the Prognosis of Outcome in Endocrine Therapy of Breast Cancer

A diagnostic assay relating diverse biomarkers to real-world, clinical, outcomes from endocrine therapy of breast cancer having being built, optimised and exercised by The present invention as hereinafter explained, a specific palette of molecular markers, also called biomarkers, useful in predicting outcomes to endocrine therapy in the treatment of breast cancer patients has been identified.

The preferred predictive palette was derived from a multivariate mathematical model where over 50 biomarkers were taken into consideration, and where seven (7) such biomarkers were found to be of improved prognostic significance taken in combination. Specifically, the most preferred palette of biomarkers predictive of outcome in endocrine therapy for breast cancer include ER, PGR, BCL2, ERBB2, MYC, KI-67, and TP-53. Another preferred predictive pallette has all of these except KI-67.

Pair-wise, as well as multivariate, dependence of certain biomarkers

Second, in accordance with the present invention an interdependency of certain biomarkers, and groups of biomarkers, has been recognised. Historically at least one dependency has been suggested. Namely, by anecdotal or better evidence it has been known that the unitary predictive value of the ER biomarker is influenced by the presence of the PGR biomarker. However, the present invention reveals new interdependencies, and even usefully quantifies these dependencies in graphs that show the varying predictive value of one biomarker in consideration of another.

Specifically for one example, the negative ER (ER-) biomarker, taken alone and without consideration of any other biomarker(s) has a certain predictive value for, specifically, the projection of percentage Disease-Specific Survival (%DSS) from 0-70+ months after commencement of endocrine therapy for patients with breast cancer. However, both the accuracy of and, happenstantially, the magnitude of, the predicted %DSS slightly increase if (1) a positive ER biomarker (BR+) is considered relative to (2) a low BCL2 (score = 0-2) logically ORed with a negative PGR (PGR-). Moreover, both the predictive accuracy and the %DSS are still yet again better if (1) a positive BR (ER+) occurs relative to (2) a high BCL2 (score - 3) logically EXCLUSIVELY ORed (XOR) with a positive PGR (PGR+). And, one of the best predictive accuracies of all, which prediction is also for an increased %DSS, occurs when (1) a positive ER (ER+) is considered relative to (2) a high BCL2 (score = 3) logically ANDed with a positive PGR (PGR+).

Specifically for yet another example, this same ER biomarker has a slightly different month-to-month %DSS predictive profile when (1) positive ER (ER+) is considered with respect to negative ERBB2 (ERBB2-); (2) negative ER (ER-) is considered with respect to positive ERBB2 (ERBB2-) or (3) positive ER (ER+) is considered with respect to negative ERBB2 (ERBB2-). However, a greatly better predictive accuracy is obtained if (4) positive ER (ER+) is considered with respect to positive ERBB2 (ERBB2+).

Finally specifically, the combination of low TP-53 (percentage of positively staining cells <70%) and high BCL2 (score = 3) has a greater percentage disease-specific survival (%DSS) than does the combination of high TP-53 (percentage of positively staining cells >=70%) and low BCL2 (score=0-2).

Now these correlations, and all of them, and still others, are reflected in the optimised mathematical model in accordance with the present invention relating (1) biomarkers to (2) the outcome of endocrine therapy on breast cancer patients. And, after just explaining in section 1 that a preferred predictive palette of biomarkers consists of no less than six different biomarkers (including all those discussed above), what, exactly, is the point of identifying that it constitutes a second aspect of the present invention that pairs of biomarkers can be related, certain pairs proving to have greater correlative association than others? The point is simply this: once it is recognised in accordance with the present invention that certain biomarkers are in stronger correlative relationship to certain others than is common among and between all biomarkers, then these biomarker "pairs" present likely fruitful avenues for investigation. For example, consider the second example above. It may be possible for an astute reader to surmise why a scientist should be interested in investigating and considering the effects, and biochemical pathways, of positive ER (ER+) in consideration of positive ERBB2 (ERBB2+). That is, PGR is a hormone receptor just as is ER. And EGFR is a growth factor receptor just as is ERBB2. Indeed, biomarkers ER (which is within the core palette of combinatory high predictive value) and EGFR (which is not within the preferred palette) were also analysed. But only positive ER (ER+) is strongly dependent upon positive ERBB2 (ERBB2+) for predictive accuracy.

The %DSS curves of two of the identified predictive biomarkers, and the close analysis thereof as a basis of recognising or confirming disease pathology

Once a certain biomarker, possibly previously suggested or even identified to be of univariate predictive significance, are suddenly in accordance with the present invention identified to be of pairwise and/or multivariate predictive significance, certain useful information can be immediately derived just by "looking hard" at the percent Disease-Specific Survival (%DSS) curves of the patient population having these biomarker characteristics. Namely, and by way of example, consider the biomarker TP-53 discussed (in conjunction with BCL2) in the immediately preceding section 2. Now the percentage of cells staining positively for TP-53, and the TP-53 score correlated with each other in individual patients. Although any amount of TP-53 staining typically is indicative of the presence of a mutant form, a sudden and significant decrease in survival was observed in patients with the highest intensity/overall score, as compared to those with but weak or moderate values (e.g., 5-year DSS was 82-86% when TP-53 intensity was 0-2 and only 53% when the intensity was 3).

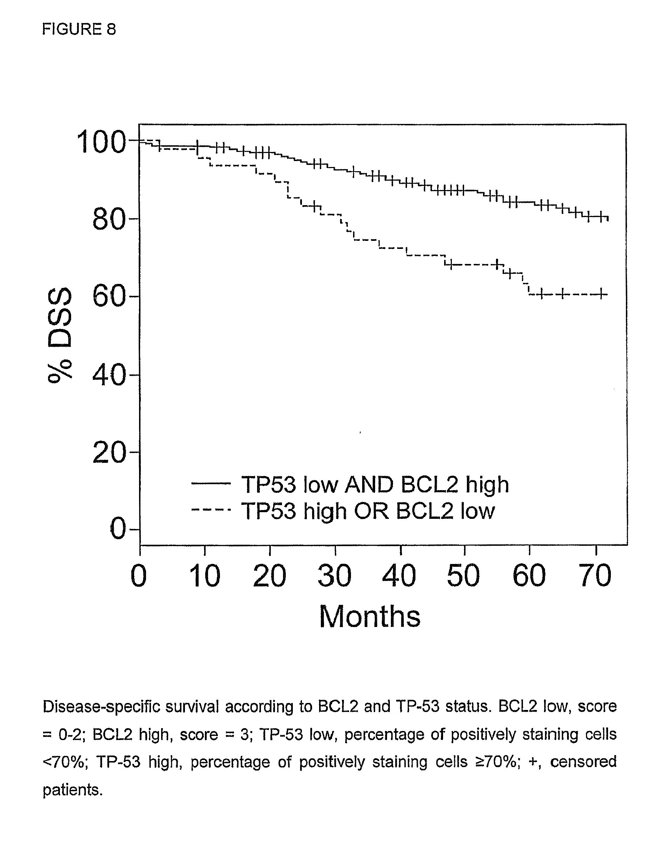

Now this merits of at least two determinations. Based on analysis of all TP-53 staining parameters, the inventors have determined that 70% positively staining cells was the most useful cut-off. This threshold between "low" and "high" TP-53 is the one used in the discussion of the relationship between TP-53 and BCL2 in the above section, and in Figure 8 of this specification. Even this much of a relation, and a determination, is useful.

But there remains the roughly 30% different survival in real patients whose cells commence to stain with high intensity for TP-53. Whether suggesting a change in treatment modalities, or simply recognising that such an occurrence is a strongly negative prognosis, the capability to usefully recognise new relationships like this is provided by the mathematically-based analysis of the present invention.

Use of an algorithm in combining the effects of several markers to predict response to therapy.

Provided in the present invention is a method of providing a treatment decision for a cancer patient receiving an endocrine therapy comprising obtaining a sample from the patient; and determining various molecular marker levels of interest in the sample, inputting such values into an algorithm which has previously correlated in a machine-learning fashion relationships between said molecular marker levels and clinical outcome, wherein output from such an algorithm indicates that that cancer is endocrine therapy resistant.

Thus, in certain embodiments of the methods of the present invention, a plurality of markers and clinicopathological factors are combined using an algorithm to increase the predictive value of the analysis in comparison to that obtained from the markers taken individually or in smaller groups. Most preferably, one or more markers for adhesion, angiogenesis, apoptosis, catenin, catenin/cadherin proliferation/differentiation, cell cycle, cell-cell interactions, cell-cell movement, cell-cell recognition, cell-cell signalling, cell surface, centrosomal, cytoskeletal, ERBB2 interaction, growth factors, growth factor receptors, invasion, metastasis, membrane/integrin, oncogenes, proliferation, tumour suppression, signal transduction, surface antigen, transcription factors and specific and non-specific markers of breast cancer are combined in a single assay to enhance the predictive value of the described methods. This assay is usefully predictive of multiple outcomes, for instance: diagnosis of breast cancer, then predicting breast cancer prognosis, then further predicting response to treatment outcome. Moreover, different marker combinations in the assay may be used for different indications. Correspondingly, different algorithms interpret the marker levels as indicated on the same assay for different indications.

In preferred embodiments, particular thresholds for one or more molecular markers in a panel are not relied upon to determine if a profile of marker levels obtained from a subject are indicative of a particular diagnosis/prognosis. Rather, in accordance with the present invention, an evaluation of the entire profile is made by (1) first training an algorithm with marker information from samples from a test population and a disease population to which the clinical outcome of interest has occurred to determine weighting factors for each marker, and (2) then evaluating that result on a previously unseen population. Certain persons skilled in bioinformatics will recognise this procedure to be tantamount to the construction, and to the training, of a neural network. The evaluation is determined by maximising the numerical area under the ROC curve for the sensitivity of a particular panel of markers versus specificity for said panel at various individual marker levels. From this number, the skilled artisan can then predict a probability that a subject's current marker levels in said combination is indicative of the clinical marker of interest. For example, (1) the test population might consist solely of samples from a group of subjects who have had ischemic stroke and no other comorbid disease conditions, while (2) the disease population might consist solely of samples from a group of subjects who have had hemorrhagic stroke and no other comorbid disease conditions. A third, "normal" population might also be used to establish baseline levels of markers as well in a non-diseased population.

In preferred embodiments of the marker, and marker panel, selection methods of the present invention, the aforementioned weighting factors are multiplicative of marker levels in a non-linear fashion. Each weighting factor is a function of other marker levels in the panel combination, and consists of terms that relate individual contributions, or independent and correlative, or dependent, terms. In the case of a marker having no interaction with other markers in regards to then clinical outcome of interest, then the specific value of the dependent terms would be zero.

Other embodiments of the instant invention

In another embodiment of the instant invention, the response to therapy is a complete pathological response.

In a preferred embodiment, the subject is a human patient.

If the tumor is breast cancer, it can, for example, be invasive breast cancer, or stage II or stage III breast cancer.

In a specific embodiment of the invention, the patient is not receiving an endocrine therapy, a chemotherapy or a hormonal therapy. In another embodiment, the patient is concurrently receiving an endocrine therapy, chemotherapy or a hormonal therapy. In a specific embodiment, the endocrine therapy comprises tamoxifen, raloxifene, megestrol, or toremifene. In a further specific embodiment, the aromatase inhibitor is anastrozole, letrozole, or exemestane, or pure anti-estrogens such fulvestrant, or surgical or medical means (goserelin, leuprolide) for reducing ovarian function. In a further specific embodiment, the cancer comprises an estrogen receptor-positive cancer or a progesterone receptor-positive cancer.

In a particular embodiment, the chemotherapy is adjuvant chemotherapy.

In another embodiment, the chemotherapy is neoadjuvant chemotherapy.

The neoadjuvant chemotherapy may, for example, comprise the administration of a taxane derivative, such as docetaxel and/or paclitaxel, and/or other anti-cancer agents, such as, members of the anthracycline class of anti-cancer agents, doxorubicin, topoisomerase inhibitors, etc.

The method may involve determination of the expression levels of at least two, or at least three, or at least four, or at least 5, or at least 6, or at least 7, or at least 8, or at least 9, or at least 10, or at least 15, or at least 20 of the prognostic proteins listed within this specification, listed above, or their associative protein expression products.

The biological sample may be e.g. a tissue sample comprising cancer cells, where the tissue can be fixed, paraffin-embedded, or fresh, or frozen.

In a particular embodiment, the tissue is from fine needle, core, or other types of biopsy.

In another embodiment, the tissue sample is obtained by fine needle aspiration, bronchial lavage, or transbronchial biopsy.

The expression level of said prognostic protein levels or associated protein levels can be determined, for example, by immunohistochemistry or a western blot, or other proteomics techniques, or any other methods known in the art, or their combination.

In an embodiment, the assay for the measurement of said prognostic proteins or their associated expression products is provided is provided in the form of a kit or kits for staining of individual proteins upon sections of tumor tissue.

In another embodiment, said kit is designed to work on an automated platform for analysis of cells and tissues such as described in U.S. patent application 10/ 062308 entitled 'Systems and methods for automated analysis of cells and tissues'.

An embodiment of the invention is a method of screening for a compound that improves the effectiveness of an endocrine therapy in a patient comprising the steps of: introducing to a cell a test agent, wherein the cell comprises polynucleotide(s) mentioned in the instant invention encoding polypeptide(s) under control of a promoter operable in the cell; and measuring said polypeptide level(s), wherein when the level(s) are decreased following the introduction, the test agent is the compound that improves effectiveness of the endocrine therapy in the patient. It is also contemplated that such an agent will prevent the development of endocrine therapy resistance in a patient receiving such a therapy. In a specific embodiment, the patient is endocrine therapy-resistant. In a further specific embodiment, the endocrine therapy comprises an adjuvant. It is also contemplated that the compound is a ribozyme, an antisense nucleotide, a receptor blocking antibody, a small molecule inhibitor, or a promoter inhibitor.

An embodiment of the invention is a method of screening for a compound that improves the effectiveness of an endocrine therapy in a patient comprising the steps of: contacting a test agent with polypeptide(s) mentioned in the instant invention, wherein said polypeptide(s) or the ER polypeptide is linked to a marker; and determining the ability of the test agent to interfere with the binding of said polypeptide(s), wherein when the marker level(s) are decreased following the contacting, the test agent is the compound that improves effectiveness of the endocrine therapy in the patient. In certain embodiments of the invention, the patient is endocrine therapy-resistant.

One embodiment of the invention is a method of treating a cancer patient comprising administering to the patient a therapeutically effective amount of an antagonist of polypeptide(s) mentioned in the instant invention and an endocrine therapy. In certain embodiments of the invention, the patient is endocrine therapy-resistant. A specific embodiment of the invention is presented wherein the antagonist interferes with translation of the polypeptide(s) mentioned in the instant invention. In a further specific embodiment of the invention the antagonist interferes with an interaction between the polypeptide(s) mentioned in the instant invention and an estrogen receptor polypeptide. The antagonist interferes with phosphorylation or any other posttranslational modification of the said polypeptide(s) in yet another specific embodiment of the invention. In another specific embodiment of the invention, the antagonist inhibits the function of a polypeptide encoding a kinase that specifically phosphorylates said polypeptide(s). In another embodiment, the antagonist is administered before, together with, or after the endocrine therapy. The antagonist and the endocrine therapy are administered at the same time in another embodiment.

An embodiment of the invention is method of improving the effectiveness of an endocrine therapy in a cancer patient comprising administering a therapeutically effective amount of an antagonist of polypeptide level (s) mentioned in the instant invention to the patient to provide a therapeutic benefit to the patient. In a specific embodiment, the administering is systemic, regional, local or direct with respect to the cancer.

An embodiment of the invention is a method of treating a cancer patient comprising: identifying an antagonist of polypeptide(s) mentioned in the instant invention by introducing to a cell a test agent, wherein the cell comprises a polynucleotide encoding a polypeptide(s) mentioned in the instant invention under control of a promoter operable in the cell, and measuring the AlB1 polypeptide level, wherein when the level is decreased following the introduction, the test agent is the antagonist of the said polypeptide(s); and administering to the patient a therapeutically effective amount of the antagonist. In certain embodiments of the invention, the patient is endocrine therapy-resistant.

An embodiment of the invention is a method of determining whether a pre-menopausal breast cancer patient should have ovariectomy as a treatment option (also goserulin, leupitine, letrozole, exesmestane, anastrozole, fulvestrant). Elevated levels of polypeptide(s) mentioned in the instant invention in a tumor sample are indicative of ovariectomy as a possible treatment option.

An embodiment of the invention is a method of determining whether a cancer patient has de novo endocrine therapy resistance comprising the steps of: obtaining a sample from the patient; and determining polypeptide(s) mentioned in the instant invention in the sample and a HER-2 polypeptide level in the sample, wherein elevated polypeptide(s) mentioned in the instant invention as compared to a control sample indicate de novo endocrine therapy resistance.

Other embodiments, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

The present invention, in particular, relates to the following:
1. A method of predicting response to endocrine therapy or predicting disease progression in breast cancer CHARACTERIZED IN THAT
   a breast cancer test sample is obtained from a subject;
   clinicopathological data is obtained from said breast cancer test sample;
   the obtained breast cancer test sample is analyzed for presence or amount of (1) one or more molecular markers of hormone receptor status, one or more growth factor receptor markers, and one or more tumor suppression/apoptosis molecular markers; (2) one or more additional molecular markers both proteomic and non-proteomic that are indicative of breast cancer disease processes from a group consisting essentially of angiogenesis, apoptosis, catenin/cadherin proliferation/differentiation, cell cycle processes, cell surface processes, cell-cell interaction, cell migration, centrosomal processes, cellular adhesion, cellular proliferation, cellular metastasis, invasion, cytoskeletal processes, ERBB2 interactions, estrogen co-receptors, growth factors and receptors, membrane/integrin/signal transduction, metastasis, oncogenes, proliferation, proliferation oncogenes, signal transduction, surface antigens and transcription factor molecular markers; and then
   (1) a presence or amount of said molecular markers is correlated with (2) clinicopathological data from said tissue sample other than the molecular markers of breast cancer disease processes, in order to deduce a probability of response to endocrine therapy or future risk of disease progression in breast cancer for the subject.
2. The method according to item 1 FURTHER CHARACTERIZED IN THAT
   the correlation is in order to deduce a probability of response to a specific endocrine therapy drawn from the group consisting of tamoxifen, anastrozole, letrozole or exemestane.
3. The method according to item 1 FURTHER CHARACTERIZED IN THAT the correlation includes
   determining the expression levels or mass spectrometry peak levels or mass-to-charge ratio(s) of one or more proteomic marker(s) and the numerical quantity of one or more clinicopathological marker(s) from breast cancer test sample excised from a patient population P1 before therapeutic treatment, clinical outcome C1 after a certain time period on said patient population P1 not known in advance; and
   comparing said determined levels and numerical values to another set of expression levels or mass spectrometry peak levels or mass-to-charge ratio(s) of one or more proteomic marker(s) and the numerical quantity of one or more clinicopathological marker(s) from breast cancer test sample excised from a separate patient population P2 before therapeutic treatment, clinical outcome C2 after said certain time period on said patient population P2 known in advance;
   wherein the clinical outcome C1 and C2 is drawn from the group consisting essentially of: breast cancer disease diagnosis, disease prognosis, or treatment outcome or a combination of any two, three or four of these outcomes; and
   training an algorithm to identify characteristic expression levels or mass spectrometry peak levels or mass-to-charge ratio(s) of one or more proteomic marker(s) and numerical quantity(ies) of one or more clinicopathological marker(s) between said patient population P1 and patient population P2 which correlate to clinical outcome C1 and clinical outcome C2, respectively.
4. The method according to item 3 FURTHER CHARACTERIZED IN THAT the training of the algorithm on characteristic protein levels or patterns of differences includes the steps of
   obtaining numerous examples of (i) said expression levels or mass spectrometry peak levels or mass-to-charge ratio(s) of one or more proteomic marker(s) and numerical quantity(ies) of one or more clinicopathological marker(s) data, and (ii) historical clinical results corresponding to this proteomic marker(s) and clinicopathological marker(s) data;
   constructing an algorithm suitable to map (i) said characteristic proteomic and said clinicopathological marker(s) data values as inputs to the algorithm, to (ii) the historical clinical results as outputs of the algorithm;
   exercising the constructed algorithm to so map (i) the said protein expression levels or mass spectrometry peak or mass-to-charge ratio(s) and clinicopathological marker(s) values as inputs to (ii) the historical clinical results as outputs; and
   conducting an automated procedure to vary the mapping function inputs to outputs, of the constructed and exercised algorithm in order that, by minimizing an error measure of the mapping function, a more optimal algorithm mapping architecture is realized;
   wherein realization of the more optimal algorithm mapping architecture, also known as feature selection, means that any irrelevant inputs are effectively excised, meaning that the more optimally mapping algorithm will substantially ignore specific proteomic marker(s) and specific clinicopathological marker(s) values that are irrelevant to output clinical results; and
   wherein realization of the more optimal algorithm mapping architecture, also known as feature selection, also means that any relevant inputs are effectively identified, making that the more optimally mapping algorithm will serve to identify, and use, those input protein expression levels or mass spectrometry peak or mass-to-charge ratio(s) and said clinicopathological marker(s) values that are relevant, in combination, to output clinical results that would result in a clinical detection of disease, disease diagnosis, disease prognosis, or treatment outcome or a combination of any two, three or four of these actions.
5. The method according to item 4 FURTHER CHARACTERIZED IN THAT the constructed algorithm is drawn from the group consisting essentially of:
   linear or nonlinear regression algorithms; linear or nonlinear classification algorithms; ANOVA; neural network algorithms; genetic algorithms; support vector machines algorithms; hierarchical analysis or clustering algorithms; hierarchical algorithms using decision trees; kernel based machine algorithms such as kernel partial least squares algorithms, kernel matching pursuit algorithms, kernel fisher discriminate analysis algorithms, or kernel principal components analysis algorithms; Bayesian probability function algorithms; Markov Blanket algorithms; a plurality of algorithms arranged in a committee network; and forward floating search or backward floating search algorithms.
6. The method according to item 4 FURTHER CHARACTERIZED IN THAT the feature selection process employs an algorithm drawn from the group consisting essentially of:
   linear or nonlinear regression algorithms; linear or nonlinear classification algorithms; ANOVA; neural network algorithms; genetic algorithms; support vector machines algorithms; hierarchical analysis or clustering algorithms; hierarchical algorithms using decision trees; kernel based machine algorithms such as kernel partial least squares algorithms, kernel matching pursuit algorithms, kernel fisher discriminate analysis algorithms, or kernel principal components analysis algorithms; Bayesian probability function algorithms; Markov Blanket algorithms; recursive feature elimination or entropy-based recursive feature elimination algorithms; a plurality of algorithms arranged in a committee network; and forward floating search or backward floating search algorithms.
7. The method according to item 4 FURTHER CHARACTERIZED IN THAT
   a tree algorithm is trained to reproduce the performance of another machine-learning classifier or regressor by enumerating the input space of said classifier or regressor to form a plurality of training examples sufficient (1) to span the input space of said classifier or regressor and (2) train the tree to emulate the performance of said classifier or regressor.
8. The method according to item 2 FURTHER CHARACTERIZED IN THAT
   the correlation to predict the response to endocrine therapy or disease progression is particularly so as to predict the response to tamoxifen or tumor aggressiveness respectively; and
   breast cancer in a patient is diagnosed by taking a biopsy of breast cancer tissue and identifying that said biopsy is wholly or partially malignant, identifying clinicopathological values associated with said malignant biopsy, analyzing said malignant tissue for the proteomic markers ER, TP-53, EEBR2, BCL-2, and one or more additional proteomic markers, evaluating the patient's prediction of response of said tumor to said therapy or evaluated risk of disease progression, respectively from said measured levels of proteomic markers and clinicopathological values, and administering tamoxifen or other therapy as appropriate to the evaluated prediction of response of said tumor to said therapy or evaluated risk of disease progression, respectively.
9. The method according to item 8 FURTHER CHARACTERIZED IN THAT the one or more additional proteomic markers analyzed includes, in addition to markers ER, TP-53, EEBR2, and BCL-2, the proteomic markers PGR, MYC, and KI67.
10. The method according to item 8 FURTHER CHARACTERIZED IN THAT the analysis one or more additional proteomic markers analyzed includes, in addition to markers ER, TP-53, EEBR2, and BCL-2, a proteomic marker of endocrine co-regulation.
11. The method of item 1 FURTHER CHARACTERIZED IN THAT
   the analyzing of one or more additional markers of breast cancer disease processes includes, in addition to one or more molecular markers of hormone receptor status, one or more growth factor receptor markers, and one or more tumor suppression molecular markers is of one or more markers selected from the group consisting of two or more of the following: ESR1, PGR, ACTC, AlB1, ANGPT1, AURKA, AURKB, BCL-2, CAV1, CCND1, CCNE, CD44, CDH1, CDH3, CDKN1B, COX2, CTNNA1, CTNNB1, CTSD, EGFR, ERBB2, ERBB2-ALT, ERBB3, ERBB4, EGFR, FGF2, FGFR1, FHIT, GATA3, GATA4, KRT14, KRT5/6, KRT8/18, KRT17, KRT19, MET, MKI67, MLLT4, MME, MMP9, MSN, MTA1, MUC1, MYC, NME1, NRG1, PARK2, PLAU, P-27, S100, SCRIB, TACC1, TACC2, TACC3, THBS1, TIMP1, TP-53, VEGF, VIM or markers related thereto.
12. The method of item 11 FURTHER CHARACTERIZED IN THAT
   the correlation is further so as to determine breast cancer treatment response or prognostic outcome; and
   the correlation is performed in accordance with an algorithm drawn from the group consisting essentially of: linear or nonlinear regression algorithms; linear or nonlinear classification algorithms; ANOVA; neural network algorithms; genetic algorithms; support vector machines algorithms; hierarchical analysis or clustering algorithms; hierarchical algorithms using decision trees; kernel based machine algorithms such as kernel partial least squares algorithms, kernel matching pursuit algorithms, kernel fisher discriminate analysis algorithms, or kernel principal components analysis algorithms; Bayesian probability function algorithms; Markov Blanket algorithms; recursive feature elimination or entropy-based recursive feature elimination algorithms; a plurality of algorithms arranged in a committee network; and forward floating search or backward floating search algorithms.
13. The method of item 12 FURTHER CHARACTERIZED IN THAT
   the correlation is to further determine treatment outcome is, in addition to prediction of response to endocrine therapy, expanded so as to predict a response to chemotherapy.
14. The method of item 1 FURTHER CHARACTERIZED IN THAT
   the correlation is of clinicopathological data selected from a group consisting of tumor nodal status, tumor grade, tumor size, tumor location, patient age, previous personal and/or familial history of breast cancer, previous personal and/or familial history of response to breast cancer therapy, and BRCA1&2 status.
15. The method of item 1 FURTHER CHARACTERIZED IN THAT
   the analysis is of both proteomic and clinicopathological markers; and
   the correlation is further so as to a clinical detection of disease, disease diagnosis, disease prognosis, or treatment outcome or a combination of any two, three or four of these actions.
16. The method of item 1 FURTHER CHARACTERIZED IN THAT
   the test sample obtained from the subject is selected from a group consisting of fixed, paraffin-embedded tissue, breast cancer tissue biopsy, tissue microarray, fresh tumor tissue, fine needle aspirates, peritoneal fluid, ductal lavage and pleural fluid or a derivative thereof.
17. The method of item 1 FURTHER CHARACTERIZED IN THAT
   the test sample is obtained from the subject before treatment of symptoms by a specific therapy; and
   the correlation is between (1) proteomic and clinicopathological marker values, and (2) the probability of present or future risk of a breast cancer progression for the subject or treatment outcome for said specific therapy, for a time period measured from the obtaining of said test sample chosen from the group consisting essentially of: 6, 12, 18, 24, 36, 60, 84, 120, or 180 months.
18. The method of item 1 FURTHER CHARACTERIZED IN THAT
   the correlation is in accordance with an algorithm drawn from the group consisting essentially of: linear or nonlinear regression algorithms; linear or nonlinear classification algorithms; ANOVA; neural network algorithms; genetic algorithms; support vector machines algorithms; hierarchical analysis or clustering algorithms; hierarchical algorithms using decision trees; kernel based machine algorithms such as kernel partial least squares algorithms, kernel matching pursuit algorithms, kernel fisher discriminate analysis algorithms, or kernel principal components analysis algorithms; Bayesian probability function algorithms; Markov Blanket algorithms; recursive feature elimination or entropy-based recursive feature elimination algorithms; a plurality of algorithms arranged in a committee network; and forward floating search or backward floating search algorithms.
19. The method of item 1 FURTHER CHARACTERIZED IN THAT
   the additional one or more molecular marker(s) includes MYC; and
   the correlation to predict the response to endocrine therapy or disease progression is of risk of breast cancer progression as given by a likelihood score derived from using Kaplan-Meier survival curves.
20. A pair of molecular markers, each of which has two conditions, suitably assessed in combination to predict the outcome in endocrine therapy for breast cancer, the molecular marker pair CHARACTERIZED IN CONSISTING ESSENTIALLY OF
   TP-53, having both a low condition defined as a percentage of positively staining cells <70% and a high condition defined as a percentage of positively staining cells >=70%; and
   BCL2 having both a high condition with a score =3, and a low condition with a score of 1 or 2.
21. The molecular marker pair of item 20 FURTHER CHARACTERIZED IN CONSISTING ESSENTIALLY OF
   ER, having both a minus condition ER- defined as absence and a positive condition ER+ defined as presence; and
   ERBB2, having both a minus condition ERBB2- defined as absence and a positive condition ERBB2+ defined as presence.
22. The molecular marker pair of item 20 FURTHER CHARACTERIZED IN CONSISTING ESSENTIALLY OF
   ER, having both a minus condition ER- defined as absence and a positive condition ER+ defined as presence; and
   ERBB2, having both a minus condition ERBB2- defined as absence and a positive condition ERBB2+ defined as presence;
   wherein the four combinations of (1) ER+ and ERBB2+, (2) ER+ and ERBB2-, (3) ER- and ERBB2+, and (4) ER- and ERBB2-, each predict a different percentage disease specific survival.
23. The molecular marker pair of item 20 FURTHER CHARACTERIZED IN CONSISTING ESSENTIALLY OF
   a first group consisting of ER, having both a minus condition ER-defined as absence and a positive condition ER+ defined as presence; and
   a second group consisting of any of
   BCL2 low, defined as a score of 0 to 2, logically ORed with PGR-, defined as absence of PGR,
   BCL2 high, defined as a score of 3, logically XORed with PGR+, defined as presence of PGR, and
   BCL2 high logically ANDed with PGR+.,
   wherein the four combinations of (1) ER-, (2) ER+ and (BCL low OR PGR-), (3) ER+ and (BCL3 high XOR PGR+), and (4) ER+ and (BCL2 high AND PGR+), each predict a different percentage disease specific survival.
24. The molecular marker pair of item 20 FURTHER CHARACTERIZED IN THAT
   the molecular marker TP-53 considered to be low if a percentage of positively staining cells is <70%, and to be high if a percentage of positively staining cells is >=70%.
25. A kit CHARACTERIZED IN THAT are present
   a panel of antibodies whose binding with breast cancer tumor samples has been correlated with breast cancer treatment outcome or patient prognosis;
   reagents to assist antibodies of said panel of antibodies in binding to tumor samples; and
   a computer algorithm, residing on a computer, operating, in consideration of all antibodies of the panel historically analyzed to bind to tumor samples, to interpolate, from the aggregation of all specific antibodies of the panel found bound to the breast cancer tumor sample, a prediction of treatment outcome for a specific treatment for breast cancer or a future risk of breast cancer progression for the subject.
26. The kit according to item 25 FURTHER CHARACTERIZED IN THAT the panel of antibodies includes
   a poly- or monoclonal antibody specific for an individual protein or protein fragment and that binds one of said antibodies correlated with breast cancer treatment outcome or patient prognosis.
27. The kit according to item 25 FURTHER CHARACTERIZED IN THAT is also present a number of immunohistochemistry assays equal to the number of antibodies within the panel of antibodies.
28. The kit according to item 25 FURTHER CHARACTERIZED IN THAT the antibodies of the panel of antibodies include
   antibodies correlated with breast cancer treatment outcome;
   and wherein the computer algorithm includes
   an algorithm using kernel partial least squares.
29. The kit according to item 25 FURTHER CHARACTERIZED IN THAT the antibodies include
   antibodies specific to ER, PGR, ERBB2, TP-53, BCL-2, KI-67 and MYC.
30. The kit according to item 25 FURTHER CHARACTERIZED IN THAT the treatment outcome predicted includes:
   response to endocrine therapy or chemotherapy.
31. The kit according to item 25 FURTHER CHARACTERIZED IN THAT the antibodies of the panel of antibodies include
   antibodies correlated with breast cancer progression; and
   wherein the computer algorithm includes:
   an algorithm using kernel partial least squares.
32. The kit according to item 25 FURTHER CHARACTERIZED IN THAT the antibodies of the panel of antibodies include
   antibodies specific to ER, ERBB2, TP-53, BCL-2, KI-67 and p-27.
33. The kit according to item 25 FURTHER CHARACTERIZED IN THAT the antibodies of the panel of antibodies include
   antibodies specific to ER, PGR, BCL-2 and ERBB2; with one or more additional markers selected from the group consisting of TP-53, KI-67, and KRT5/6; and with one or more additional markers selected from the group consisting of TP-53, KI-67, and KRT5/6; and with one or more additional markers selected from the group consisting of MSN, C-MYC, CAV1, CTNNB1, CDH1, MME, AURKA, P-27, GATA3, HER4, VEGF, CTNNA1, and CCNE.

### BRIEF DESCRIPTION OF THE DRAWINGS

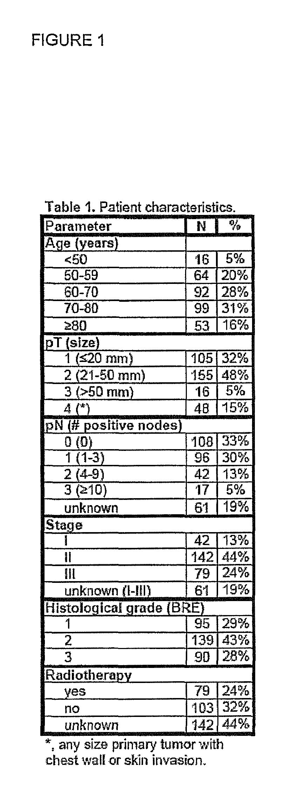

Figure 1 is Table 1 of patient characteristics.

Figure 2 is Table 2 of Univariate Cox Proportional hazard analysis of clinicopathological features for five-year survival.

Figure 3 is Table 3 of Univariate Cox Proportional hazard analysis of molecular makers (biomarkers) for five-year survival.

Figure 4 is Table 4 of Cox Proportional hazard analysis of conditional molecular makers (biomarkers) for five-year survival.

Figure 5 is Table 5 of Multivariate Cox Proportional hazard analysis of conditional molecular makers (biomarkers) for five-year survival.

Figure 6 is a graph of the percentage disease-specific survival rate (%DSS) from 0 to 70+ months according to the status of ER, PGR and BCL2 biomarkers.

Figure 7 is a graph of the percentage disease-specific survival rate (%DSS) from 0 to 70+ months according to the status of ER and BRBB2 biomarkers.

Figure 8 is a graph of the percentage disease-specific survival rate (%DSS) from 0 to 70+ months according to the status of BCL2 and TP-53 biomarkers.

Figure 9 is a graph showing ROC curves demonstrating the prognostic accuracy of the NPI vs. the multi-marker model.

Figure 10 is a graph showing Kaplan-Meier survival curves comparing multi-marker and NPI models.

Figure 11 is a table showing area under the operating receiver curve for various prognostic indicators of tamoxifen resistance.

### DETAILED DESCRIPTION OF THE INVENTION

Definitions

As used herein, the term "adjuvant" refers to a pharmacological agent that is provided to a patient as an additional therapy to the primary treatment of a disease or condition.

The term "algorithm" as used herein refers to a mathematical formula that provides a relationship between two or more quantities. Such a formula may be linear, non-linear, and may exist as various numerical weighting factors in computer memory.

The term "interaction of two or more markers" refers to an interaction that is functional or productive. Such an interaction may lead to downstream signaling events. Other contemplated interactions allow further productive binding events with other molecules.

The term "control sample" as used herein indicates a sample that is compared to a patient sample. A control sample may be obtained from the same tissue that the patient sample is taken from. However, a noncancerous area may be chosen to reflect the individual polypeptide levels in normal cells for a particular patient. A control may be a cell line, such as MCF-7, in which serial dilutions are undertaken to determine the exact concentration of elevated polypeptide levels. Such levels are compared with a patient sample. A "control sample" may comprise a theoretical patient with an elevated polypeptide level of a certain molecule that is calculated to be the cutoff point for elevated polypeptide levels of said certain molecule. A patient sample that has polypeptide levels equal to or greater than such a control sample is said to have elevated polypeptide levels.

As used herein, the term "overall survival" is defined to be survival after first diagnosis and death. For instance, long-term overall survival is for at least 5 years, more preferably for at least 8 years, most preferably for at least 10 years following surgery or other treatment.

The term "disease-free survival" as used herein is defined as a time between the first diagnosis and/or first surgery to treat a cancer patient and a first reoccurrence. For example, a disease-free survival is "low" if the cancer patient has a first reoccurrence within five years after tumor resection, and more specifically, if the cancer patient has less than about 55% disease-free survival over 5 years. For example, a high disease-free survival refers to at least about 55% disease-free survival over 5 years.

The term "endocrine therapy" as used herein is defined as a treatment of or pertaining to any of the ducts or endocrine glands characterized by secreting internally and into the bloodstream from the cells of the gland. The treatment may remove the gland, block hormone synthesis, or prevent the hormone from binding to its receptor.

The term "endocrine therapy-resistant patient" as used herein is defined as a patient receiving an endocrine therapy and lacks demonstration of a desired physiological effect, such as a therapeutic benefit, from the administration of an endocrine therapy.

The term "estrogen-receptor positive" as used herein refers to cancers that do have estrogen receptors while those breast cancers that do not possess estrogen receptors are "estrogen receptor-negative."

The term "polypeptide" as used herein is used interchangeably with the term "protein", and is defined as a molecule which comprises more than one amino acid subunits. The polypeptide may be an entire protein or it may be a fragment of a protein, such as a peptide or an oligopeptide. The polypeptide may also comprise alterations to the amino acid subunits, such as methylation or acetylation. The term "molecular marker" is also used interchangeably with the terms protein and polypeptide, though the two latter terms are subclasses of the former.

The term "prediction" is used herein to refer to the likelihood that a patient will respond either favorably or unfavorably to a drug or set of drugs, and also the extent of those responses, or that a patient will survive, following surgical removal or the primary tumor and/or chemotherapy for a certain period of time without cancer recurrence. The predictive methods of the present invention are valuable tools in predicting if a patient is likely to respond favorably to a treatment regimen, such as surgical intervention, chemotherapy with a given drug or drug combination, and/or radiation therapy, or whether long-term survival of the patient, following surgery and/or termination of chemotherapy or other treatment modalities is likely.

The term "prognosis" as used herein are defined as a prediction of a probable course and/or outcome of a disease. For example, in the present invention the combination of several protein levels together with an interpolative algorithm constitutes a prognostic model for resistance to endocrine therapy in a cancer patient.

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

The term "therapeutic benefit" as used herein refers to anything that promotes or enhances the well-being of the subject with respect to the medical treatment of his condition, which includes treatment of pre-cancer, cancer, and hyperproliferative diseases. A list of nonexhaustive examples of this includes extension of the subject's life by any period of time, decrease or delay in the neoplastic development of the disease, decrease in hyperproliferation, reduction in tumor growth, delay of metastases, reduction in cancer cell or tumor cell proliferation rate, and a decrease in pain to the subject that can be attributed to the subject's condition. In a specific embodiment, a therapeutic benefit refers to reversing de novo endocrine therapy-resistance or preventing the patient from acquiring an endocrine therapy-resistance.

The term "treatment" as used herein is defined as the management of a patient through medical or surgical means. The treatment improves or alleviates at least one symptom of a medical condition or disease and is not required to provide a cure. The term "treatment outcome" as used herein is the physical effect upon the patient of the treatment.

The term "sample" as used herein indicates a patient sample containing at least one tumor cell. Tissue or cell samples can be removed from almost any part of the body. The most appropriate method for obtaining a sample depends on the type of cancer that is suspected or diagnosed. Biopsy methods include needle, endoscopic, and excisional. The treatment of the tumor sample after removal from the body depends on the type of detection method that will be employed for determining individual protein levels.

Detailed Description

Most existing statistical and computational methods for biomarker identification of disease states, disease prognosis, or treatment outcome, such as U.S. Patent applications 10/331,127 and/or U.S. patent application 10/883,303, have focused on differential expression of markers between diseased and control data sets. This metric is tested by simple calculation of fold changes, by t-test, and/or F test. These are based on variations of linear discriminant analysis (i.e., calculating some or the entire covariance matrix between features).

However, the majority of these data analysis methods are not effective for biomarker identification and disease diagnosis for the following reasons. First, although the calculation of fold changes or t-test and F-test can identify highly differentially expressed biomarkers, the classification accuracy of identified biomarkers by these methods, is, in general, not very high. This is because linear transforms typically extract information from only the second- order correlations in the data (the covariance matrix) and ignore higher-order correlations in the data. We have shown that proteomic datasets are inherently non-symmetric (See for Instance Linke et al Clin. Can. Research Feb. 15, 2006). For such cases, non-linear transforms are necessary. Second, most scoring methods do not use classification accuracy to measure a biomarker's ability to discriminate between classes. Therefore, biomarkers that are ranked according to these scores may not achieve the highest classification accuracy among biomarkers in the experiments. Even if some scoring methods, which are based on classification methods, are able to identify biomarkers with high classification accuracy among all biomarkers in the experiments, the classification accuracy of a single marker cannot achieve the required accuracy in clinical diagnosis. Third, a simple combination of highly ranked markers according to their scores or discrimination ability is usually not be efficient for classification, as shown in the instant invention, If there is high mutual correlation between markers, then complexity increases without much gain.

Accordingly, the instant invention provides a methodology that can be used for biomarker feature selection and classification, and is applied in the instant application to prognosis of breast cancer and endocrine treatment outcome.

Exemplary Biomarkers related to prognosis of breast cancer and endocrine treatment outcome.

A comprehensive methodology for identification of one or more markers for the prognosis, diagnosis, and detection of disease has been described previously. Suitable methods for identifying such diagnostic, prognostic, or disease-detecting markers are described in detail in U.S. patent 6,658,396, NEURAL NETWORK DRUG DOSAGE ESTIMATION, U.S. Patent Application No. 09/611,220, entitled NEURAL-NETWORK-BASED INDENTIFICATION, AND APPLICATION, OF GENOMIC INFORMATION PRACTICALLY RELEVANT TO DIVERSE BIOLOGICAL AND SOCIOLOGICAL PROBLEMS, filed July 6, 2000, and U.S. provisional patent application Ser. No. 10/948,834, entitled DIAGNOSTIC MARKERS OF CARDIOVASCULAR ILLNESS AND METHODS OF USE THEREOF, filed Sept. 23, 2003, each of which patents and parent applications is hereby incorporated by reference in its entirety, including all tables, figures, and claims. Briefly, our method of predicting relevant markers given an individual's test sample is an automated technique of constructing an optimal mapping between a given set of input marker data and a given clinical variable of interest. We illustrate this method further in the following section called "Methodology of Marker Selection, Analysis, and Classification"

We first obtain patient test samples of tissue from two or more groups of patients. The patients are those exhibiting symptoms of a disease event, say breast cancer, and who are prescribed a specific therapeutic treatment which has a specific clinical outcome are compared to a different set of patients also exhibiting the same disease event but with different therapeutic treatments and/or clinical outcome of said treatment. These second sets of patients are viewed as controls, though these patients might have another disease event distinct from the first. Samples from these patients are taken at various time periods after the event has occurred, and assayed for various markers as described within. Clinicopathological information, such as age, tumor stage, tumor histological grade, and node status are collected at time of diagnosis. These markers and clinicopathological information form a set of examples of clinical inputs and their corresponding outputs, the outputs being the clinical outcome of interest, for instance breast cancer prognosis and/or breast cancer therapeutic treatment outcome.

We then use an algorithm to select the most relevant clinical inputs that correspond to the outcome for each time period. This process is also known as feature selection. In this process, the minimum number of relevant clinical inputs that are needed to fully differentiate and/or predict disease prognosis, diagnosis, or detection with the highest sensitivity and specificity are selected for each time period. The feature selection is done with an algorithm that selects markers that differentiate between patient disease groups, say those likely to have recurrence versus those likely to no recurrence. The relevant clinical input combinations might change at different time periods, and might be different for different clinical outcomes of interest.

We then train a classifier to map the selected relevant clinical inputs to the outputs. A classifier assigns relative weightings to individual marker values. We note that the construct of a classifier is not crucial to our method. Any mapping procedure between inputs and outputs that produces a measure of goodness of fit, for example, maximizing the area under the receiver operator curve of sensitivity versus 1-specificity, for the training data and maximizes it with a standard optimization routine on a series of validation sets would also suffice.

Once the classifier is trained, it is ready for use by a clinician. The clinician enters the same classifier inputs used during training of the network by assaying the selected markers and collecting relevant clinical information for a new patient, and the trained classifier outputs a maximum likelihood estimator for the value of the output given the inputs for the current patient. The clinician or patient can then act on this value. We note that a straightforward extension of our technique could produce an optimum range of output values given the patient's inputs as well as specific threshold values for inputs.

One versed in the ordinary state of the art knows that many other polypeptides in the literature once measured from tumor tissue in a diseased patient and healthy tissue from a healthy patient, selected through use of an feature selection algorithm might be prognostic of breast cancer or breast cancer treatment outcome if measured in combination with others and evaluated together with a nonlinear classification algorithm. We have described some of these other polypeptides, previously considered for diagnosis or prognosis of breast cancer and thus not novel in themselves in an accompanying U.S. patent application with the same title as this PCT application. That list is meant to serve as illustrative and not meant to be exhaustive. Selected polypeptide descriptions in the U.S. patent application list may be similar to U.S. patent application 10/758307, U.S. patent application 11/061,067 and/or U.S. patent application 10/872,063, all of which are noted as prior art. However, the instant invention goes beyond what is taught or anticipated in these applications, providing a rigorous methodology of discovering which representative polypeptides are best suited to building a predictive model for determining a clinical outcome and building a model for interpolating between such polypeptides in conjunction with clinicopathological variables to determine clinical outcome, while the methodology described in U.S. patent application 10/758307, U.S. patent application 11/061,067 and/or U.S. patent application 10/872063 rely on simple linear relationships between markers and linear optimization techniques to find them. Using such techniques, the instant invention also defines smaller, more robust sets of polypeptides that are more predictive of clinical outcome than what is described or anticipated in such applications.

Method for Defining Panels of Markers

In practice, data may be obtained from a group of subjects. The subjects may be patients who have been tested for the presence or level of certain polypeptides and/or clinicopathological variables (hereafter 'markers' or 'biomarkers'). Such markers and methods of patient extraction are well known to those skilled in the art. A particular set of markers may be relevant to a particular condition or disease. The method is not dependent on the actual markers. The markers discussed in this document are included only for illustration and are not intended to limit the scope of the invention. Examples of such markers and panels of markers are described above in the instant invention and the incorporated references.

Well-known to one of ordinary skill in the art is the collection of patient samples. A preferred embodiment of the instant invention is that the samples come from two or more different sets of patients, one a disease group of interest and the other(s) a control group, which may be healthy or diseased in a different indication than the disease group of interest. For instance, one might want to look at the difference in markers between patients who have had endocrine therapy and had a recurrence of cancer within a certain time period and those who had endocrine therapy and did not have recurrence of cancer within the same time period to differentiate between the two populations.

When obtaining tumor samples for testing according to the present invention, it is generally preferred that the samples represent or reflect characteristics of a population of patients or samples. It may also be useful to handle and process the samples under conditions and according to techniques common to clinical laboratories. Although the present invention is not intended to be limited to the strategies used for processing tumor samples, we note that, in the field of pathology, it is often common to fix samples in buffered formalin, and then to dehydrate them by immersion in increasing concentrations of ethanol followed by xylene.. Samples are then embedded into paraffin, which is then molded into a "paraffin block" that is a standard intermediate in histologic processing of tissue samples. The present inventors have found that many useful antibodies to biomarkers discussed herein display comparable binding regardless of the method of preparation of tumor samples; those of ordinary skill in the art can readily adjust observations to account for differences in preparation procedure.

In preferred embodiments of the invention, large numbers of tissue samples are analyzed simultaneously. In some embodiments, a tissue array is prepared. Tissue arrays may be constructed according to a variety of techniques. According to one procedure, a commercially-available mechanical device (e.g., the manual tissue arrayer MTA1 from Beecher Instruments of Sun Prairie, Wis.) is used to remove an 0.6-micron-diameter, full thickness "core" from a paraffin block (the donor block) prepared from each patient, and to insert the core into a separate paraffin block (the recipient block) in a designated location on a grid. In preferred embodiments, cores from as many as about 400 patients can be inserted into a single recipient block; preferably, core-to-core spacing is approximately 1 mm. The resulting tissue array may be processed into thin sections for staining with interaction partners according to standard methods applicable to paraffin embedded material. Depending upon the thickness of the donor blocks, as well as the dimensions of the clinical material, a single tissue array can yield about 50-150 slides containing >75% relevant tumor material for assessment with interaction partners. Construction of two or more parallel tissue arrays of cores from the same cohort of patient samples can provide relevant tumor material from the same set of patients in duplicate or more. Of course, in some cases, additional samples will be present in one array and not another.

The tumor test samples are assayed by one or more techniques, well-known for those versed in ordinary skill in the art for various polypeptide levels. Briefly, assays are conducted by binding a certain substance with a detectable label to the antibody of the protein in question to be assayed and bringing such in contact with the tumor sample to be assayed. Any available technique may be used to detect binding between an interaction partner and a tumour sample. One powerful and commonly used technique is to have a detectable label associated (directly or indirectly) with the antibody. For example, commonly-used labels that often are associated with antibodies used in binding studies include fluorochromes, enzymes, gold, iodine, etc. Tissue staining by bound interaction partners is then assessed, preferably by a trained pathologist or cytotechnologist. For example, a scoring system may be utilised to designate whether the antibody to the polypeptide does or does not bind to (e.g., stain) the sample, whether it stains the sample strongly or weakly and/or whether useful information could not be obtained (e.g., because the sample was lost, there was no tumor in the sample or the result was otherwise ambiguous). Those of ordinary skill in the art will recognise that the precise characteristics of the scoring system are not critical to the invention. For example, staining may be assessed qualitatively or quantitatively; more or less subtle gradations of staining may be defined; etc.

It is to be understood that the present invention is not limited to using antibodies or antibody fragments as interaction partners of inventive tumour markers. In particular, the present invention also encompasses the use of synthetic interaction partners that mimic the functions of antibodies. Several approaches to designing and/or identifying antibody mimics have been proposed and demonstrated (e.g., see the reviews by Hsieh-Wilson et al., Acc. Chem. Res. 29:164, 2000 and Peczuh and Hamilton, Chem. Rev. 100:2479, 2000). For example, small molecules that bind protein surfaces in a fashion similar to that of natural proteins have been identified by screening synthetic libraries of small molecules or natural product isolates (e.g., see Gallop et al., J. Med. Chem. 37:1233, 1994; Gordon et al., J. Med. Chem. 37:1385, 1994)

Any available strategy or system may be utilised to detect association between an antibody and its associated polypeptide molecular marker. In certain embodiments, association can be detected by adding a detectable label to the antibody. In other embodiments, association can be detected by using a labeled secondary antibody that associates specifically with the antibody, e.g., as is well known in the art of antigen/antibody detection. The detectable label may be directly detectable or indirectly detectable, e.g., through combined action with one or more additional members of a signal producing system. Examples of directly detectable labels include radioactive, paramagnetic, fluorescent, light scattering, absorptive and calorimetric labels. Examples of indirectly detectable include chemiluminescent labels, e.g., enzymes that are capable of converting a substrate to a chromogenic product such as alkaline phosphatase, horseradish peroxidase and the like.

Once a labeled antibody has bound a tumor marker, the complex may be visualized or detected in a variety of ways, with the particular manner of detection being chosen based on the particular detectable label, where representative detection means include, e.g., scintillation counting, autoradiography, measurement of paramagnetism, fluorescence measurement, light absorption measurement, measurement of light scattering and the like.

In general, association between an antibody and its polypeptide molecular marker may be assayed by contacting the antibody with a tumor sample that includes the marker. Depending upon the nature of the sample, appropriate methods include, but are not limited to, immunohistochemistry (IHC), radioimmunoassay, ELISA, immunoblotting and fluorescence activates cell sorting (FACS). In the case where the polypeptide is to be detected in a tissue sample, e.g., a biopsy sample, 1HC is a particularly appropriate detection method. Techniques for obtaining tissue and cell samples and performing IHC and FACS are well known in the art.

In general, the results of such an assay can be presented in any of a variety of formats. The results can be presented in a qualitative fashion. For example, the test report may indicate only whether or not a particular protein biomarker was detected, perhaps also with an indication of the limits of detection. Additionally the test report may indicate the subcellular location of binding, e.g., nuclear versus cytoplasmic and/or the relative levels of binding in these different subcellular locations. The results may be presented in a semi-quantitative fashion. For example, various ranges may be defined and the ranges may be assigned a score (e.g., 0 to 5) that provides a certain degree of quantitative information. Such a score may reflect various factors, e.g., the number of cells in which the tumor marker is detected, the intensity of the signal (which may indicate the level of expression of the tumor marker), etc. The results may be presented in a quantitative fashion, e.g., as a percentage of cells in which the tumor marker is detected, as a concentration, etc. As will be appreciated by one of ordinary skill in the art, the type of output provided by a test will vary depending upon the technical limitations of the test and the biological significance associated with detection of the protein biomarker. For example, in the case of certain protein biomarkers a purely qualitative output (e.g., whether or not the protein is detected at a certain detection level) provides significant information. In other cases a more quantitative output (e.g., a ratio of the level of expression of the protein in two samples) is necessary.

The resulting set of values are put into a database, along with outcome, also called phenotype, information detailing the treatment type, for instance tamoxifen plus chemotherapy, once this is known. Additional patient or tumour test sample details such as patient nodal status, histological grade, cancer stage, the sum total called patient clinicopathological information, are put into the database. The database can be simple as a spreadsheet, i.e. a two-dimensional table of values, with rows being patients and columns being filled with patient marker and other characteristic values.

From this database, a computerized algorithm can first perform preprocessing of the data values. This involves normalisation of the values across the dataset and/or transformation into a different representation for further processing. The dataset is then analysed for missing values. Missing values are either replaced using an imputation algorithm, in a preferred embodiment using KNN or MVC algorithms, or the patient attached to the missing value is excised from the database. If greater than 50% of the other patients have the same missing value then value can be ignored.

Once all missing values have been accounted for, the dataset is split up into three parts: a training set comprising 33-80% of the patients and their associated values, a testing set comprising 10-50% of the patients and their associated values, and a validation set comprising 1-50% of the patients and their associated values. These datasets can be further sub-divided or combined according to algorithmic accuracy. A feature selection algorithm is applied to the training dataset. This feature selection algorithm selects the most relevant marker values and/or patient characteristics. Preferred feature selection algorithms include, but are not limited to, Forward or Backward Floating, SVMs, Markov Blankets, Tree Based Methods with node discarding, Genetic Algorithms, Regression-based methods, kernel-based methods, and filter-based methods.

Feature selection is done in a cross-validated fashion, preferably in a naïve or k-fold fashion, as to not induce bias in the results and is tested with the testing dataset. Cross-validation is one of several approaches to estimating how well the features selected from some training data is going to perform on future as-yet-unseen data and is well-known to the skilled artisan. Cross validation is a model evaluation method that is better than residuals. The problem with residual evaluations is that they do not give an indication of how well the learner will do when it is asked to make new predictions for data it has not already seen. One way to overcome this problem is to not use the entire data set when training a learner. Some of the data is removed before training begins. Then when training is done, the data that was removed can be used to test the performance of the learned model on "new" data.

Once the algorithm has returned a list of selected markers, one can optimize these selected markers by applying a classifier to the training dataset to predict clinical outcome. A cost function that the classifier optimizes is specified according to outcome desired, for instance an area under receiver-operator curve maximising the product of sensitivity and specificity of the selected markers, or positive or negative predictive accuracy. Testing of the classifier is done on the testing dataset in a cross-validated fashion, preferably naïve or k-fold cross-validation. Further detail is given in U.S. patent application 09/611,220, incorporated by reference. Classifiers map input variables, in this case patient marker values, to outcomes of interest, for instance, prediction of stroke sub-type. Preferred classifiers include, but are not limited to, neural networks, Decision Trees, genetic algorithms, SVMs, Regression Trees, Cascade Correlation, Group Method Data Handling (GMDH), Multivariate Adaptive Regression Splines (MARS), Multilinear Interpolation, Radial Basis Functions, Robust Regression, Cascade Correlation + Projection Pursuit, linear regression, Non-linear regression, Polynomial Regression, Regression Trees, Multilinear Interpolation, MARS, Bayes classifiers and networks, and Markov Models, and Kernel Methods.

The classification model is then optimised by for instance combining the model with other models in an ensemble fashion. Preferred methods for classifier optimization include, but are not limited to, boosting, bagging, entropy-based, and voting networks. This classifier is now known as the final predictive model. The predictive model is tested on the validation data set, not used in either feature selection or classification, to obtain an estimate of performance in a similar population.

The predictive model can be translated into a decision tree format for subdividing the patient population and making the decision output of the model easy to understand for the clinician. The marker input values might include a time since symptom onset value and/or a threshold value. Using these marker inputs, the predictive model delivers diagnostic or prognostic output value along with associated error. The instant invention anticipates a kit comprised of reagents, devices and instructions for performing the assays, and a computer software program comprised of the predictive model that interprets the assay values when entered into the predictive model run on a computer. The predictive model receives the marker values via the computer that it resides upon.

Once patients are exhibiting symptoms of cancer, for instance breast cancer, a tissue tumor sample is taken from the patient using standard techniques well known to those of ordinary skill in the art and assayed for various tumor markers of cancer by slicing it along its radial axis and placing such slices upon a substrate for molecular analysis by assaying for various molecular markers. Assays can be preformed through immunohistochemistry or through any of the other techniques well known to the skilled artisan. In a preferred embodiment, the assay is in a format that permits multiple markers to be tested from one sample, such as the Aqua platform.TM., and/or in a quantitative fashion, defined to within 10% of the actual value and in the most preferred enablement of the instant invention, within 1 % of the actual value. The values of the markers in the samples are inputted into the trained, tested, and validated algorithm residing on a computer, which outputs to the user on a display and/or in printed format on paper and/or transmits the information to another display source the result of the algorithm calculations in numerical form, a probability estimate of the clinical diagnosis of the patient. There is an error given to the probability estimate, in a preferred embodiment this error level is a confidence level. The medical worker can then use this diagnosis to help guide treatment of the patient.

In another embodiment, the present invention provides a kit for the analysis of markers. Such a kit preferably comprises devises and reagents for the analysis of at least one test sample and instructions for performing the assay. Optionally the kits may contain one or more means for using information obtained from immunoassays performed for a marker panel to rule in or out certain diagnoses. Marker antibodies or antigens may be incorporated into immunoassay diagnostic kits depending upon which marker autoantibodies or antigens are being measured. A first container may include a composition comprising an antigen or antibody preparation. Both antibody and antigen preparations should preferably be provided in a suitable titrated form, with antigen concentrations and/or antibody titers given for easy reference in quantitative applications.

The kits may also include an immunodetection reagent or label for the detection of specific immunoreaction between the provided antigen and/or antibody, as the case may be, and the diagnostic sample. Suitable detection reagents are well known in the art as exemplified by radioactive, enzymatic or otherwise chromogenic ligands, which are typically employed in association with the antigen and/or antibody, or in association with a second antibody having specificity for first antibody. Thus, the reaction is detected or quantified by means of detecting or quantifying the label. Immunodetection reagents and processes suitable for application in connection with the novel methods of the present invention are generally well known in the art.

The reagents may also include ancillary agents such as buffering agents and protein stabilizing agents, e.g., polysaccharides and the like. The diagnostic kit may further include where necessary agents for reducing background interference in a test, agents for increasing signal, software and algorithms for combining and interpolating marker values to produce a prediction of clinical outcome of interest, apparatus for conducting a test, calibration curves and charts, standardization curves and charts, and the like.

Various aspects of the invention may be better understood in view of the following detailed descriptions, examples, discussion, and supporting references.

### EXAMPLES

### EXAMPLE I

1. Derivation of, and Conclusions from, the Invention in brief

The clinical studies, and the mathematical analysis, leading to the present invention of (1) a mathematical model, and (2) insights resulting from exercise of the model, were entirely designed to assess the contributions of several molecular markers, in addition to standard clinicopathological data, to the prediction of tamoxifen treatment outcome and disease progression in breast cancer.

The patients and clinical methods used are as follows. The clinical study of the present invention is retrospective, and based on data from some 324 stage I-III female breast cancer patients treated with tamoxifen for whom standard clinicopathological data and tumour tissue microarrays were available. Over 50 molecular markers were studied, including ER, PGR, BCL2, CDKN1B, EGFR, ERBB2, and TP-53 expression by semi-quantitative immunohistochemistry; and also CCND1, ERBB2, and MYC gene amplification by fluorescence in situ hybridization. Cox proportional hazard analysis was used to determine the contributions of each parameter to disease-specific and overall survival.

The results of a multivariate mathematical analysis of all makers, are, as succinctly explained in language (as opposed to mathematics), as follows. On a univariate basis, high pathological tumour or nodal class, histological grade, EGFR, ERBB2, MYC, or TP-53; absent ER or PGR; and low BCL2 were significantly associated with worse survival. On a multivariate basis, nodal class, ER, and MYC were statistically significant as independent factors for survival. In addition, PGR, BCL2, and ERBB2 moderated the benefit of ER positive status, and BCL2 and TP-53 were additional significant risk factors.

The conclusion of the mathematical analysis, succinctly explained, is as follows: The data demonstrates the prognostic value of BCL-2, ERBB2, MYC, MKI67, and TP-53, in addition to the standard hormone receptors ER and PGR, clinicopathological features, in a multivariate model of tamoxifen treatment outcome. In addition, they demonstrate the importance of conditional interpretation of certain molecular markers to maximize their utility.

### EXAMPLE II

Patients and Methods Providing Input Data to the Mathematical Analysis of the Present Invention

2.1 Patient Data

Clinical, pathological, and molecular marker patient data were obtained from University Hospital in Basel, Switzerland (See for instance Torhorst J, Bucher C, Kononen J, et al: Tissue microarrays for rapid linking of molecular changes to clinical endpoints. Am J Pathol 159:2249-2256, 2001) for 324 stage I-III female breast cancer patients who received hormone therapy but no adjuvant cytotoxic chemotherapy. Tamoxifen was used in nearly all of the cases, although it is possible that a negligible number of patients (<2%) could have received a different hormone therapy. A subset of the patients received neoadjuvant cytotoxic chemotherapy (<1%) and/or adjuvant radiotherapy, but these were not statistically significant factors in survival. The patients were treated at the University Hospital in Basel (Switzerland), the Women's Hospital Rheinfelden (Germany), and the Kreiskrankenhaus Lörrach (Germany) between 1985 and 1994. Patient identities had been anonymized, and the Ethics Committee of the Basel University Clinics had approved the use of the specimens and data for research.

2.2 Immunohistochemistry (IHC)

Mouse monoclonal antibodies (clone; epitope, if applicable; dilution) against ER (1D5; N- terminus; 1:1,000), PGR (1A6; A/B region; 1:600), BCL2 (124; 1:1), CDKN1B (SX53G8; 1:1,000), EGFR (EGFR.113; extracellular domain; 1:20), and TP-53 (DO-7; N-terminus; 1:1) were used for immunohistochemical analysis. All antibodies were obtained from DAKO, except PGR and EGFR, which were obtained from Novocastra. The HercepTest kit (DAKO) was used for ERBB2. Antibodies for the other 50 markers studied were internally generated.

Tissue microarrays (TMAs) constructed from formalin-fixed paraffin-embedded primary tumour samples and were stained with a standard immunoperoxidase IHC protocol (See for instance) 15. Tumors with known positivity were used as positive controls, and the primary antibodies were eliminated for negative controls. The markers were scored for both intensity (on a scale of 0-3) and the estimated percentage of positively staining cells in approximately 10% increments. Final scores on a 0-3 scale (0 = none, 1 = weak, 2 = moderate, and 3 = strong) were determined from a combination of these attributes for these markers.

For the statistical analyses, ER and PGR were considered positive/present when staining was evident in 10% or more of cells. BCL2 was considered "high" when the final score was 3 (strong). ERBB2 staining was scored only on the intensity scale of 0-3, and it was considered "positive" when the score was greater than 0.

2.3 Fluorescence in situ hybridization (FISH)

CCND1, ERBB2, and MYC gene amplifications were determined as described elsewhere (See for instance Al-Kuraya K, Schraml P, Torhorst J, et al: Prognostic relevance of gene amplifications and coamplifications in breast cancer. Cancer Res 64:8534-8540, 2004). Briefly, the TMAs were proteolyzed, deparaffinized, dehydrated, and denatured. They were then subjected to standard dual-label FISH with Spectrum-Orange-labeled gene-specific probes and Spectrum-Green-labeled centromere probe controls from chromosomes 11 (CCND1), 17 (ERBB2), and 8 (MYC). The nuclei were counterstained with DAPI in antifade solution, and they were examined by indirect fluorescence microscopy. A gene was considered amplified if the ratio of its signal number to that of the corresponding centromere was =2.

2.4 Statistical Methods

Survival measures were defined as the proportions of patients who were still alive for a defined number of months after diagnosis. For overall survival (OS), death from any cause was included. For disease-specific survival (DSS), patients who died due to a cause other than cancer were censored. All parameters were studied with Cox proportional hazard analysis. For categorical analyses, thresholds were determined empirically by finding maximal hazard ratios. Analyses were conducted with the computer software MATLAB version R14 (The Mathworks Inc., Natick, MA), and R17 with the Survival package (See for instance R Survival. R Development Core Team, v2.15, ISBN 3-900051-07-0, http://www.R-project.org).

3. Results of the Mathematical Analysis

3.1 Patient characteristics

The clinicopathological features of the full set of 324 stage I-III breast cancer patients are shown in Table 1. Mean age at diagnosis was 64.3 years. Pathological tumour class (pT) was known for all patients and is dependent on the size or invasiveness of the primary tumour. Pathological nodal class (pN) was known for 81% of the patients and is dependent on the number of positive lymph nodes. Stage was determined by combining the pT and pN parameters using the 2002-modified American Joint Committee on Cancer staging system. Histological grade was determined by the Elston-modified Bloom/Richardson method (BRE) (See for instance Elston CW, Ellis IO: Pathological prognostic factors in breast cancer. I. The value of histological grade in breast cancer: experience from a large study with long-term follow-up. Histopathology 19:403- 410, 1991). The tumors were predominantly ductal (75%) and lobular (14%) carcinomas.

3.2 Univariate analysis of clinicopathological features

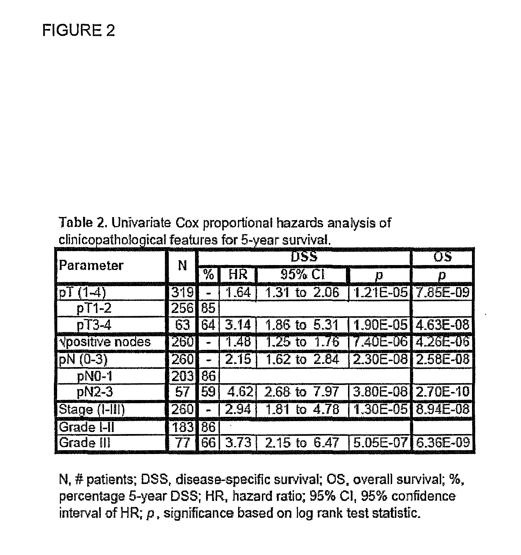

In univariate Cox proportional hazard analysis of the clinicopathological characteristics (Table 2 (Figure 2)), increasing values for pT, the square root of the number of positive nodes, pN, and stage were significantly associated with shorter survival. Patients with pT3-4, pN2-3, or histological grade III were at particularly high risk for recurrence relative to the lower classes in each case (Table 2 (Figure 2)).

3.3 Univariate analysis of molecular marker data

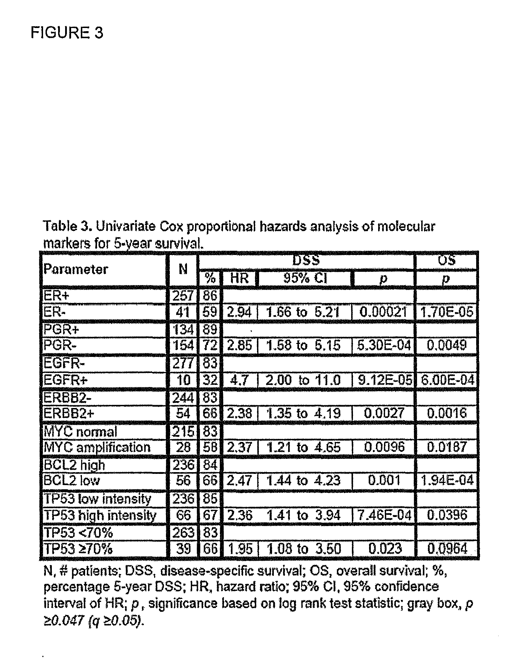

In univariate Cox proportional hazard analysis (Table 3 (Figure 3)), the lack of ER or PGR, or the presence of EGFR, ERBB2, or amplified MYC, were all significantly associated with shorter survival. Low BCL2 (all scores below the maximum of 3) also was significantly associated with shorter survival. TP-53 was significantly associated with worse outcome when the staining intensity was moderate to strong (TP-53 7 high intensity). A high percentage (=70%) of cells staining positively for TP-53 was not significant for OS and was only marginally significant for DSS in this independent univariate analysis. However, it became more significant when considering interactions with BCL2 (see below). The hazard ratios for DSS and OS were not statistically different in all cases when the covariate remained statistically significant for OS, although only the P values are shown for OS. CCND1, and CDKN1B were not significant in this analysis, and the number of EGFR-positive patients was insufficient to assess its contribution.

3.4 Analysis of molecular markers based on interactions with other factors

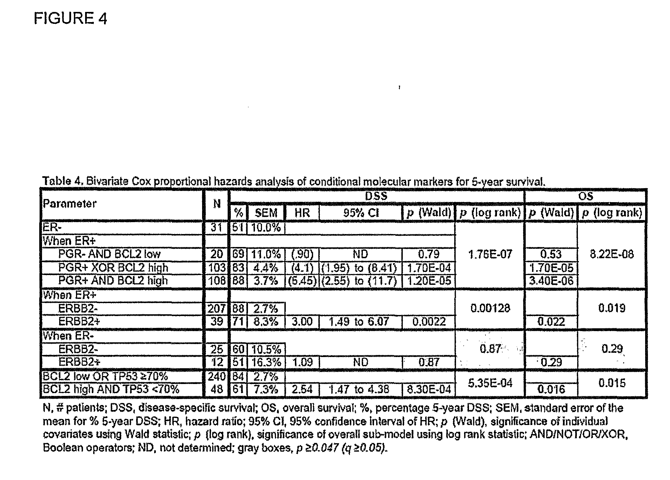

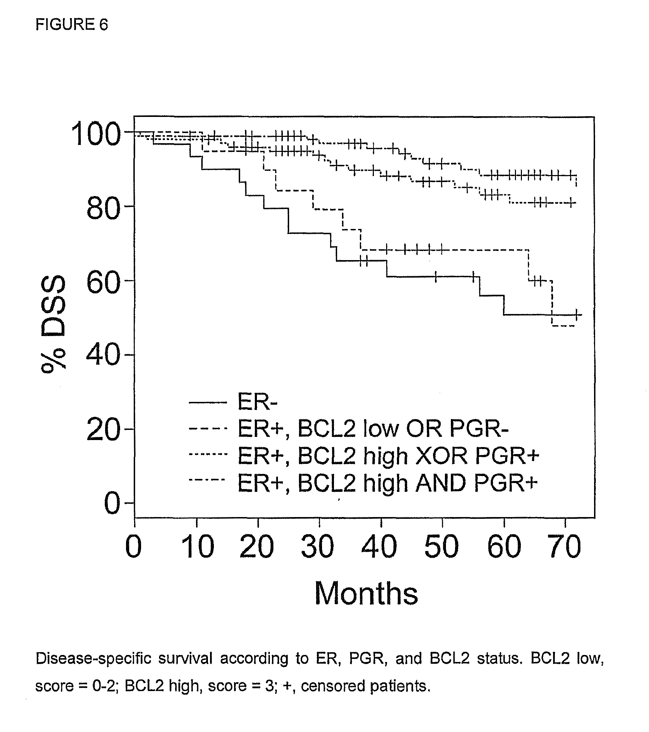

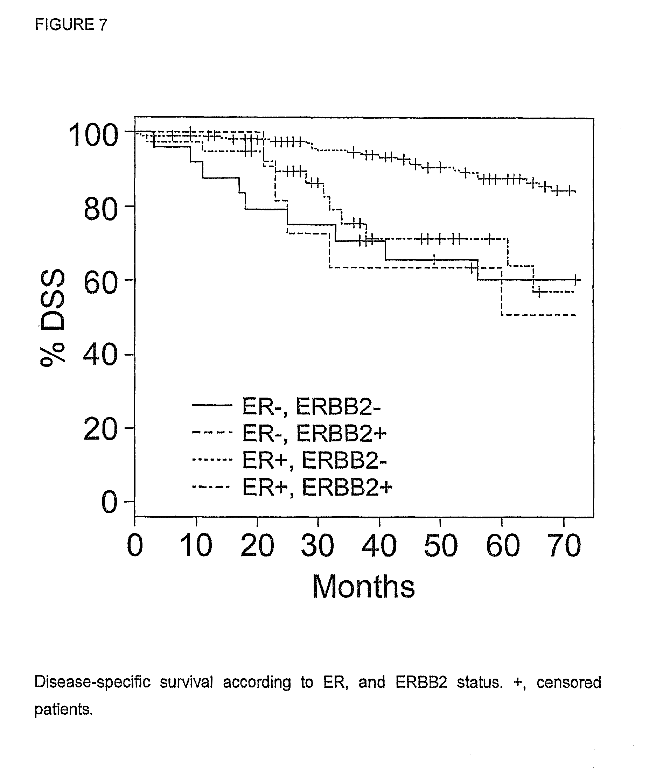

Several of the molecular markers exhibited dependencies relative to other markers, which increased their prognostic values. For example, DSS and OS of ER-positive patients who were PGR- negative and who had low BCL2 scores were not statistically different than ER-negative patients. However, if either PGR was present or the BCL2 score was high, the ER-positive patients had significantly better outcome. Independent of this observation, ER-positive patients who were both PGR- positive and BCL2-high experienced even better outcome (Table 4 (Figure 4); Figure 6). Similarly, ERBB2 positivity was significantly associated with worse outcome in ER-positive patients, but not ER-negative patients (Table 4 (Figure 4); Figure 7).

There were also strong interactions between BCL2 and TP-53. In the subset of patients with low TP-53 staining, low BCL2 staining was significantly associated with worse DSS, and in the subset of patients with high BCL2 staining, high TP-53 staining was significantly associated with worse DSS. However, when one of these markers of poorer outcome (low BCL2 or high TP-53) was evident, the status of the other marker did not significantly further affect outcome (data not shown). Combining these results, the presence of either low BCL2 or high TP-53 or both was significantly associated with worse DSS and OS (Table 4 (Figure 4); Figure 8).

Not surprisingly, values for TP-53 intensity, the percentage of cells staining positively for TP-53, and TP-53 score correlated with each other in individual patients. Although any amount of TP-53 staining typically is indicative of the presence of a mutant form, we observed a sudden and significant decrease in survival in patients with the highest intensity/overall score, as compared to those with weak or moderate values (e.g., 5-year DSS was 82-86% when TP-53 intensity was 0-2 and only 53% when the intensity was 3). Based on analysis of all TP-53 staining parameters, it was determined that 70% positively staining cells was the most useful cut-off.

### EXAMPLE III

3.5 Multivariate model

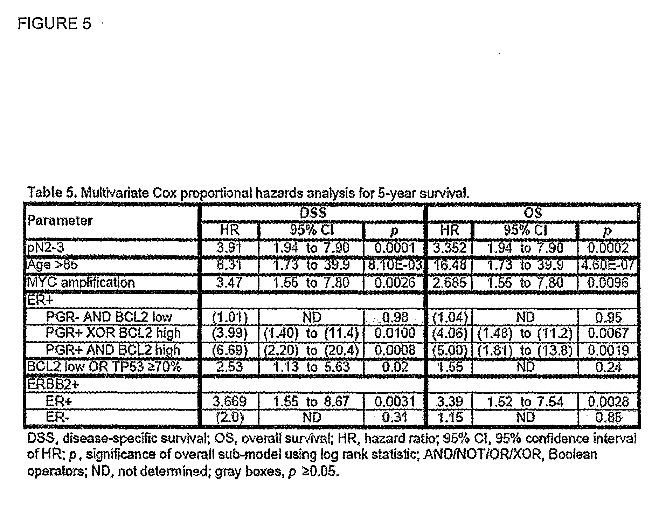

A multivariate Cox proportional hazards model was constructed based on the univariate analyses. pN, age, MYC, ER (including interactions with PGR and BCL2), BCL2 (including interaction with TP-53), and ERBB2 (including interaction with ER) remained independent for both DSS and OS (Table 5 (Figure 5)). The overall P values (log rank statistic) of the multivariate model were highly significant at 3.22E-12 and 3.58E-09, respectively, for DSS and OS.

3.6 Multi-marker model based upon machine learning- Endocrine Therapy

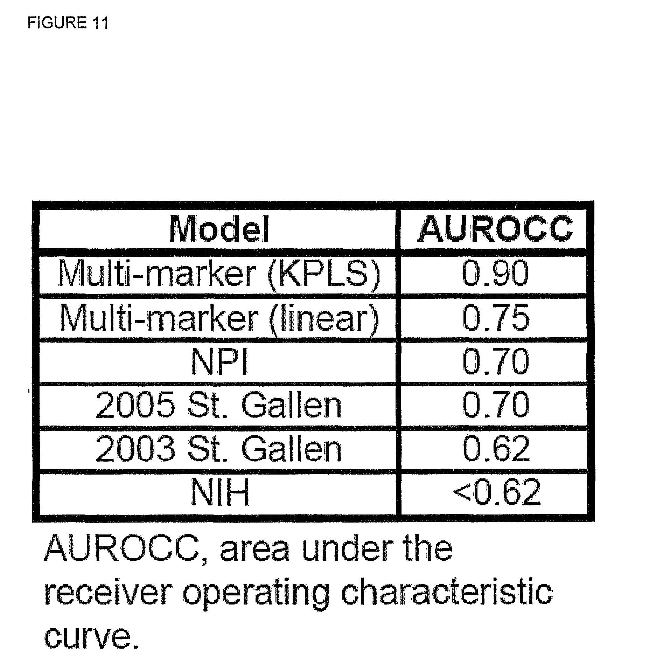

Using the features and the cutoff values contained in the multivariate model, a predictive model based on five-year RFS was produced for hormone receptor-positive patients by means of a kernel partial least squares (KPLS) third-order polynomial with four-fold/three-repeat cross-validation. One of the best ways to compare the predictive accuracy of different models is through Receiver Operating Curve (ROC) analysis. ROC curves were plotted for the multi-marker model (incorporating the NPI), as well as the NPI alone and the St. Gallen consensus guidelines. Our multi-marker model performed significantly better than the current standards (Figure 4 (Figure 4)). The area under the ROC curve was 0.90 for our model, while it was only 0.71 and 0.62 for the NPI and St. Gallen guidelines, respectively. The NIH guidelines performed slightly worse than the St. Gallen guidelines (Figure 11). Interestingly, standard linear regression modelling of our multi-marker dataset achieved an AUROCC of 0.75, indicating that both inclusion of the additional markers and our machine learning-based modelling contributed significantly to the improved performance.

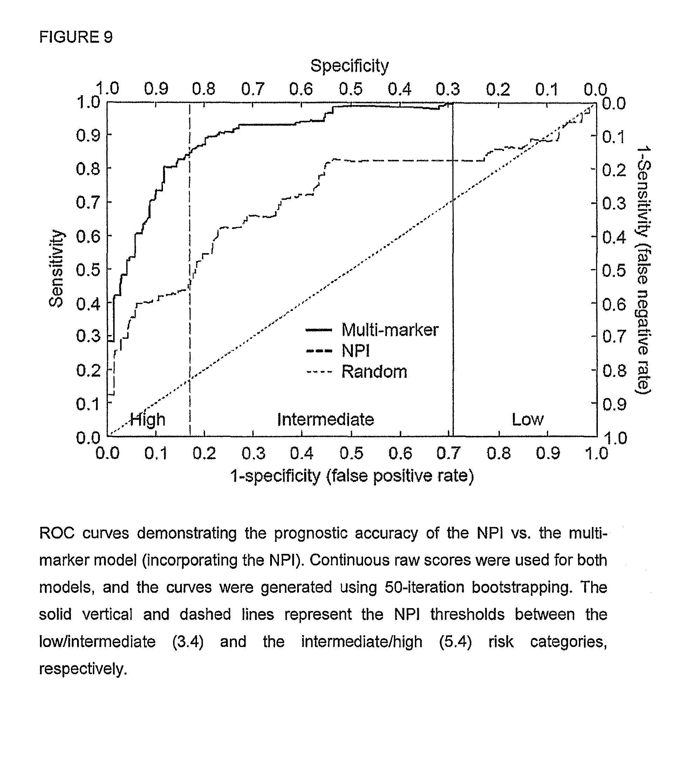

Direct comparisons between models can also be made at specific operating points on the ROC curves. Using the NPI low-risk threshold score of 3.4, the NPI identified 83% of the patients who had a recurrence within five years (sensitivity). However, it did so at the expense of incorrectly predicting recurrence in 73% of the patients who remained recurrence-free (false positive rate). In contrast, using the same 83% sensitivity rate, the multi-marker model had a false positive rate of only 15% (Figure 9).

Using the NPI high-risk threshold score of 5.4, the NPI correctly classified 82% of the patients who remained recurrence-free for five years (specificity). However, it only correctly classified 43% of the patients who had a recurrence (sensitivity). In contrast, using the same 82% specificity rate, the multi-marker model correctly classified 85% of the patients who had a recurrence (Figure 9).

The multi-marker model was dominant at all operating points on the ROC curves. For example, at the more diagnostically useful threshold of 90% specificity, the multi-marker model had a sensitivity of 73%, outperforming the NPI's 41% sensitivity. The NPI's sensitivity performance reached a maximum at 83%. At that sensitivity, the specificity of the multi-marker model is 86%, outperforming the NPI's 54% specificity. In fact, the multi-marker model continues to perform well at much higher sensitivities, producing a specificity of 72% at a sensitivity of 93%. In contrast, the specificity of the NPI falls below 10% at sensitivities greater than 90% (Figure 9).

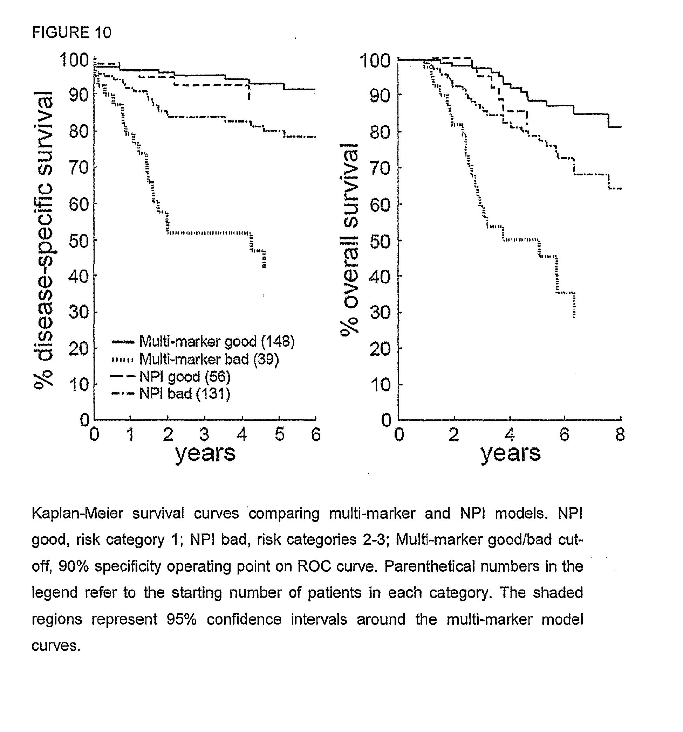

Kaplan-Meier survival analysis also revealed the superiority of the multi-marker model over the NPI when patients were categorised as having either a "good" or "poor" prognosis with tamoxifen treatment alone (Figure 10). Since chemotherapy is typically considered for NPI intermediate- and high-risk patients, they were designated as poor prognosis. A specificity of 90% was chosen as a cut-off for the multi-marker model. Although the NPI successfully categorised a subset of good prognosis patients with similar survival characteristics as the multi-marker model, the multi-marker model was able to classify significantly more patients into this category. Correspondingly, the increased accuracy of the multi-marker model in classifying the bad prognosis patients resulted in significantly shorter survival in a smaller set of patients compared to the NPI (Figure 10).

3.7 Multi-marker model based upon machine learning-Chemotherapy

The data set selected for assessment was the subset of patients whom had chemotherapy, some of which had hormone treatment as well.

The molecular marker dataset was first coded for machine learning. Missing values were given a numerical tag. A series of latent features were constructed from the raw biomarker data.

For feature selection, a 5-year survival curve was used as the objective measure. A wrapper based feature selection was used to select the biomarkers and jointly optimize model parameters. Specifically, the area under the receiver operating characteristic curve was used as the optimization function for biomarker selection. The machine learning method employed was kernel partial least squares (KPLS). During feature selection, the KPLS algorithm had the number of latent features set to 9 and used a polynomial kernel of order 3. The data set was subdivided into 5 disjoint folds to serve as naïve testing sets. Each of these had a corresponding training set. The feature selection was performed on the training set using SFFS and employing nested cross validation to scores. The SFFS algorithm was allowed to run 50 epochs. This process was repeated independently for each training fold.

Following feature selection, the best performing sets of features were rescored using 5 fold cross validation on each training fold. Based on model order number of subjects and 5X cross validation scores on the training set, a set of features were chosen from each training fold to form the ensemble model.

Each set of features was then retrained on the training fold to provide a set of trained submodels. These submodels were applied to the naïve-testing fold to provide a naive estimate of performance, such that the model estimate was an average of the results provided by the naïve submodels. Each of the feature sets were also evaluated using 5X cross-validation on the full data set. The 5X cross validation results on the training fold and on the complete data set for each of the submodels is provided in Table 1-1, and respectively in tables 2-1,3-1, 4-1, and 5-1. The sets of specific biomarkers selected for each fold for each submodel are given in Table 1-2, and respectively Tables 2-2, 3-2, 4-2 and 5-2, for the other training folds. The corresponding type of features by group are given in Table 1-3, and respectively 2-3, 3-3, 4-3, and 5-3 for the other training folds.

The performance of the models developed from the individual feature sets on the full data set, using 5 fold cross validation, on average had an AUC ROC in the mid 0.80s. The performance of the naïve results was anticipated to be slightly lower, and was found to be 0.80, 0.52, 0.72, 0.89 and 0.80 for each of the naïve testing folds, respectively.

The result of this is that 3 of the 5 folds displayed the expected degree of performance, but fold 2 did not generalize. It is notable, that of all of the training folds, fold 2 had the simplest models, which may have serendipitously well represented the selected subset of subjects, but been insufficient for model generalization. Likewise, fold 3 had lower than anticipated naïve results.

The sets of features with their individual scores are thus scored both on the training set and on the combined data set using 5X-cross validation, as well as being trained and the naively applied to the corresponding test set. They provide an indication that these features are important in the treatment outcome of subjects with breast cancer that were treated with chemotherapy and tamoxifen or other hormone therapies.

The consensus across all 5 folds in terms of features consistently selected were likely primary features consisting of HORMONE TREATMENT, BCL-2, SIZE, ER-PR, and ERBB2. All of these features appeared in at least 3 of the models as recurrent feature, or in multiple models as a recurrent feature with some inclusion in the other models. The next set of features occurred less consistently in the independent model folds, but occurred in at least 2/5 models. These features are TP-53, GRADE, PN, MKI67, and KRT5/6. Finally, there were a set of features that were observed intermittently in the selected models. MSN, C-MYC, CAV1, CTNNB1, CDH1, MME, AURKA, P-27, GATA3, HER4, VEGF, CTNNA1, and CCNE. These features are contextual, but may impact the outcome of subjects.

**Table 1-1 Performance results for Training Fold 1.**

| Submodel Number | Model Order | N Subjects | 5X CV on Train Fold | | 5X CV on All | |
|---|---|---|---|---|---|---|
| | | | Roc AUC | (std) | CV All | (std) |
| 1 | 5 | 91 | 0.89 | 0.07 | 0.83 | 0.03 |
| 2 | 5 | 95 | 0.91 | 0.04 | 0.80 | 0.07 |
| 3 | 5 | 95 | 0.92 | 0.02 | 0.84 | 0.02 |
| 4 | 6 | 75 | 0.92 | 0.02 | 0.86 | 0.03 |
| 5 | 6 | 75 | 0.94 | 0.02 | 0.82 | 0.03 |

Selected submodels with model order, the subject number with complete data. Performance is given as the cross validated area under the receiver operating characteristic curve (AUC ROC) and its corresponding standard deviation is provided for training set Fold 1. The ROC AUC evaluated using the selected features on the full data set (All Folds) is also given for reference.

**Table 1-2 Fold 1 submodel feature names**

| Submodel Number | Feature Names | | | | | |
|---|---|---|---|---|---|---|
| Submodel Number | Model Order | N Subjects | 5X CV on Train Fold | | 5X CV on All | |
| | | | Roc AUC | (std) | CV All | (std) |
| 1 | 3 | 80 | 0.92 | 0.03 | 0.79 | 0.05 |
| 2 | 3 | 84 | 0.94 | 0.03 | 0.83 | 0.02 |
| 3 | 4 | 81 | 0.93 | 0.04 | 0.85 | 0.05 |
| 4 | 5 | 82 | 0.90 | 0.03 | 0.85 | 0.06 |
| 5 | 5 | 74 | 0.91 | 0.02 | 0.82 | 0.05 |

Selected submodels with model order, the subject number with complete data. Performance is given as the cross validated area under the receiver operating characteristic curve (AUC ROC) and its corresponding standard deviation is provided for training set Fold 2. The ROC AUC evaluated using the selected features on the full data set (All Folds) is also given for reference.

**Table 2-2 Fold 2 submodel feature names**

| Submodel Number | Feature Names |
|---|---|
| 1 | TREATMENT, CTNNB1, BCL-2-QS-OLD |
| 2 | TREATMENT, BCL-2-QS-OLD, MKI67 |
| 3 | TREATMENT, BCL-2-QS-OLD, MKI67, BCL-2-PCT-NEW |
| 4 | BCL-2-QS-OLD-HIGH-STATUS, TREATMENT, MKI67, CDH1, HR-STATUS=10 |
| 5 | TREATMENT, BCL-2-QS-OLD, MKI67, BCL-2-PCT-NEW, CTNNB1 |

**Table 2-3 Fold 2 submodel feature groups**

| Submodel Number | Feature Groups |
|---|---|
| 1 | HORMONE TREATEMENT, BLC2, CTNNB1 |
| 2 | HORMONE TREATMENT, BLC2, MKI67 |
| 3 | HORMONE TREATMENT, BLC2, MKI67 |
| 4 | HORMONE TREATMENT, BLC2, MKI67, CDH1, ER-PR |
| 5 | HORMONE TREATMENT, BLC2, MKI67, CTNNB1 |

**Table 3-1 Performance results for Training Fold 3.**

| Submodel Number | Model Order | N Subjects | 5X CV on Train Fold | | 5X CV on All | |
|---|---|---|---|---|---|---|
| | | | Roc AUC | (std) | CV All | (std) |
| 1 | 5 | 86 | 0.95 | 0.02 | 0.85 | 0.02 |
| 2 | 4 | 90 | 0.93 | 0.03 | 0.79 | 0.04 |
| 3 | 5 | 89 | 0.92 | 0.05 | 0.84 | 0.08 |
| 4 | 5 | 89 | 0.93 | 0.05 | 0.83 | 0.09 |

Selected submodels with model order, the subject number with complete data. Performance is given as the cross validated area under the receiver operating characteristic curve (AUC ROC) and its corresponding standard deviation is provided for training set Fold 3. The ROC AUC evaluated using the selected features on the full data set (All Folds) is also given for reference.

**Table 3-2 Fold 3 submodel feature names**

| Submodel Number | Feature Names |
|---|---|
| 1 | TREATMENT, BCL-2-QS-OLD, N+CODE, AURKA, SIZE |
| 2 | ERBB4, GRADE, ERBB2-STATUS-2, PR |
| 3 | SIZE, ERBB4, ERBB2-STATUS-2, GRADE, ER-10-STATUS |
| 4 | ERBB4, GRADE, HER2ERPOS, SIZE, ERBB2-STATUS-2 |

**Table 3-3 Fold 3 submodel feature groups**

| Submodel Number | Feature Groups |
|---|---|
| 1 | HORMONE TREATEMENT, BLC2, SIZE, AURKA |
| 2 | ERBB4, GRADE, ERBB2, ER-PR |
| 3 | SIZE, ERBB4, ERBB2, GRADE, ER-PR |
| 4 | SIZE, ERBB4, ERBB2, GRADE, ER-PR |

**Table 4-1 Performance results for Training Fold 4.**

| Submodel Number | Model Order | N Subjects | 5X CV on Train Fold | | 5X CV on All | |
|---|---|---|---|---|---|---|
| | | | Roc AUC | (std) | CV All | (std) |
| 1 | 4 | 81 | 0.92 | 0.04 | 0.78 | 0.06 |
| 2 | 4 | 80 | 0.91 | 0.04 | 0.80 | 0.03 |
| 3 | 5 | 76 | 0.92 | 0.02 | 0.86 | 0.04 |
| 4 | 5 | 80 | 0.92 | 0.03 | 0.82 | 0.06 |
| 5 | 6 | 80 | 0.94 | 0.01 | 0.89 | 0.04 |
| 6 | 6 | 80 | 0.94 | 0.02 | 0.82 | 0.04 |

Selected submodels with model order, the subject number with complete data. Performance is given as the cross validated area under the receiver operating characteristic curve (AUC ROC) and its corresponding standard deviation is provided for training set Fold 4. The ROC AUC evaluated using the selected features on the full data set (All Folds) is also given for reference.

**Table 4-2 Fold 4 submodel feature names**

| Submodel Number | Feature Names |
|---|---|
| 1 | HORMONE, KRT5/6, PN, CDKN1B |
| 2 | ERBB24, BCL-2-QS-OLD-HIGH-STATUS, BCL-2-RES-NEW-LOW-STATUS, PN |
| 3 | BCL-2-QS-MEAN, PT, KRT5/6, TREATMENT, BCL-2-QS-OLD |
| 4 | ERBB4, BCL-2-QS-OLD-HIGH-STATUS, BCL-2-RES-NEW-LOW-STATUS, PN, ERBB2-STATUS-1 |
| 5 | GRADE, GATA3, MYC-IHC-STATUS, HR-STATUS>0, MKI67, BCL-2-INT-NEW |
| 6 | ERBB4, BCL-2-QS-OLD-HIGH-STATUS, BCL-2-RES-NEW-LOW-STATUS, PN, ERBB2-STATUS-1, BCL-2-QS-MEAN |

**Table 4-3 Fold 4 submodel feature groups**

| Submodel Number | Feature Groups |
|---|---|
| 1 | HORMONE TREATEMENT, KRT5/6, PN, CDKN1B |
| 2 | BCL-2, ERBB4, PN |
| 3 | BCL-2, PT, KRT5/6, HORMONE TREATMENT |
| 4 | BCL-2, ERBB4, PN, ERBB2 |
| 5 | BCL-2, GRADE, GATA3, C-MYC, ER-PR, MKI67 |
| 6 | BCL-2, ERBB4, PN, ERBB2 |

**Table 5-1 Performance results for Training Fold 5.**

| Submodel Number | Model Order | N Subjects | 5X CV on Train Fold | | 5X CV on All | |
|---|---|---|---|---|---|---|
| | | | Roc AUC | (std) | CV All | (std) |
| 1 | 4 | 79 | 0.89 | 0.02 | 0.83 | 0.06 |
| 2 | 5 | 76 | 0.90 | 0.01 | 0.84 | 0.06 |
| 3 | 5 | 79 | 0.92 | 0.02 | 0.81 | 0.06 |
| 4 | 5 | 78 | 0.94 | 0.03 | 0.83 | 0.05 |
| 5 | 5 | 80 | 0.94 | 0.01 | 0.82 | 0.09 |

Selected submodels with model order, the subject number with complete data. Performance is given as the cross validated area under the receiver operating characteristic curve (AUC ROC) and its corresponding standard deviation is provided for training set Fold 5. The ROC AUC evaluated using the selected features on the full data set (All Folds) is also given for reference.

**Table 5-2 Fold 5 submodel feature names**

| Submodel Number | Feature Names |
|---|---|
| 1 | BCL-2-QS-OLD, SIZE, MKI67, CCNE |
| 2 | BCL-2-QS-OLD-HIGH-STATUS, SIZE, TP-53_HIGH, HORMONE, ER_PR.AND.BCL-2 |
| 3 | KRT5/6, BCL-2-QS-MEAN-LOW-STATUS, CHEMO, HORMONE, VEGF |
| 4 | SIZE, CTNNA1, CDKN1B, CCNE, AURKA |
| 5 | KRT5/6, BCL-2-QS-MEAN-LOW-STATUS, HORMONE, VEGF, SIZE |

**Table 5-3 Fold 5 submodel feature names**

| Submodel Number | Feature Groups |
|---|---|
| 1 | BCL-2, SIZE, MKI67, CCNE |
| 2 | BCL-2, SIZE, TP-53, HORMONE TREATMENT, ER-PR |
| 3 | BCL-2, KRT5/6, HORMONE TREATMENT, VEGF |
| 4 | SIZE, CTNNA1, CDKN1B, CCNE, AURKA |
| 5 | BCL-2, SIZE, KRT5/6, HORMONE TREATMENT, VEGF |

To produce the survival curves (Figure 5), a specific threshold value was chosen to categorize patients with "good" or "bad" prognosis with tamoxifen treatment alone. However, the multi-marker model can be used to produce a risk of recurrence percentage as a continuous function of the score, so patients and their oncologists could be provided with a more specific risk rating. Alternatively, two thresholds could be chosen, one at specificity x to recommend against more aggressive therapy, and another at sensitivity y to recommend more aggressive therapy. Although there would be an "intermediate" group of patients with ambiguous scores, this set of patients would be significantly smaller than it is with the current prognostic standards in which a majority or plurality of patients end up in that category.

### EXAMPLE IV

Evaluation of a combined data set of chemotherapy treated with and without tamoxifen therapy, tamoxifen only treatment, and no treatment groups for a low order model of disease outcome (progression) indicated that p27 p53 and blc2 formed a candidate model (524 patient data set) with an AUC ROC of approximately 0.70 and potentially up to 0.80. Evaluation of these same markers in the 324-patient data set described previously on tamoxifen treated subjects with and without chemotherapy therapy yielded a positive result. 116 subjects had complete marker and outcome data that could be assessed from the 324 patient data set. The training was performed with a kernel partial least squares model. The specifics of the markers were Quick Score on blc2 and p27 and pct on p53. Specifically, the leave one out cross validated performance assessed by the area under the receiver operator curve AUC ROC was 0.68. A similar exercise on the 524 patient data set with 5 fold cross validation with 3 repeats of the operation for a total of 15 folds also yielded an AUC ROC of 0.68 +/- 0.02

In accordance with these and still other insights obtained by the building, and the exercise, of the diagnostic test in accordance with the present invention, the invention should be broadly defined by the following claims.

## Claims

1. A method of predicting response to endocrine therapy or predicting disease progression in breast cancer **CHARACTERIZED IN THAT**
a breast cancer test sample is obtained from a subject;
clinicopathological data is obtained from said breast cancer test sample;
the obtained breast cancer test sample is analyzed for presence or amount of (1) one or more molecular markers of hormone receptor status, one or more growth factor receptor markers, and one or more tumor suppression/apoptosis molecular markers; (2) one or more additional molecular markers both proteomic and non-proteomic that are indicative of breast cancer disease processes from a group consisting essentially of angiogenesis, apoptosis, catenin/cadherin proliferation/differentiation, cell cycle processes, cell surface processes, cell-cell interaction, cell migration, centrosomal processes, cellular adhesion, cellular proliferation, cellular metastasis, invasion, cytoskeletal processes, ERBB2 interactions, estrogen co-receptors, growth factors and receptors, membrane/integrin/signal transduction, metastasis, oncogenes, proliferation, proliferation oncogenes, signal transduction, surface antigens and transcription factor molecular markers; and then
(1) a presence or amount of said molecular markers is correlated with (2) clinicopathological data from said tissue sample other than the molecular markers of breast cancer disease processes, in order to deduce a probability of response to endocrine therapy or future risk of disease progression in breast cancer for the subject.

2. The method according to claim 1 FURTHER **CHARACTERIZED IN THAT**
the correlation is in order to deduce a probability of response to a specific endocrine therapy drawn from the group consisting of tamoxifen, anastrozole, letrozole or exemestane.

3. The method according to claim 1 FURTHER **CHARACTERIZED IN THAT** the correlation includes
determining the expression levels or mass spectrometry peak levels or mass-to-charge ratio(s) of one or more proteomic marker(s) and the numerical quantity of one or more clinicopathological marker(s) from breast cancer test sample excised from a patient population P1 before therapeutic treatment, clinical outcome C1 after a certain time period on said patient population P1 not known in advance; and
comparing said determined levels and numerical values to another set of expression levels or mass spectrometry peak levels or mass-to-charge ratio(s) of one or more proteomic marker(s) and the numerical quantity of one or more clinicopathological marker(s) from breast cancer test sample excised from a separate patient population P2 before therapeutic treatment, clinical outcome C2 after said certain time period on said patient population P2 known in advance;
wherein the clinical outcome C1 and C2 is drawn from the group consisting essentially of: breast cancer disease diagnosis, disease prognosis, or treatment outcome or a combination of any two, three or four of these outcomes; and
training an algorithm to identify characteristic expression levels or mass spectrometry peak levels or mass-to-charge ratio(s) of one or more proteomic marker(s) and numerical quantity(ies) of one or more clinicopathological marker(s) between said patient population P1 and patient population P2 which correlate to clinical outcome C1 and clinical outcome C2, respectively.

4. The method according to claim 3 FURTHER **CHARACTERIZED IN THAT** the training of the algorithm on characteristic protein levels or patterns of differences includes the steps of
obtaining numerous examples of (i) said expression levels or mass spectrometry peak levels or mass-to-charge ratio(s) of one or more proteomic marker(s) and numerical quantity(ies) of one or more clinicopathological marker(s) data, and (ii) historical clinical results corresponding to this proteomic marker(s) and clinicopathological marker(s) data;
constructing an algorithm suitable to map (i) said characteristic proteomic and said clinicopathological marker(s) data values as inputs to the algorithm, to (ii) the historical clinical results as outputs of the algorithm;
exercising the constructed algorithm to so map (i) the said protein expression levels or mass spectrometry peak or mass-to-charge ratio(s) and clinicopathological marker(s) values as inputs to (ii) the historical clinical results as outputs; and
conducting an automated procedure to vary the mapping function inputs to outputs, of the constructed and exercised algorithm in order that, by minimizing an error measure of the mapping function, a more optimal algorithm mapping architecture is realized;
wherein realization of the more optimal algorithm mapping architecture, also known as feature selection, means that any irrelevant inputs are effectively excised, meaning that the more optimally mapping algorithm will substantially ignore specific proteomic marker(s) and specific clinicopathological marker(s) values that are irrelevant to output clinical results; and
wherein realization of the more optimal algorithm mapping architecture, also known as feature selection, also means that any relevant inputs are effectively identified, making that the more optimally mapping algorithm will serve to identify, and use, those input protein expression levels or mass spectrometry peak or mass-to-charge ratio(s) and said clinicopathological marker(s) values that are relevant, in combination, to output clinical results that would result in a clinical detection of disease, disease diagnosis, disease prognosis, or treatment outcome or a combination of any two, three or four of these actions.

5. The method according to claim 4 FURTHER **CHARACTERIZED IN THAT** the constructed algorithm is drawn from the group consisting essentially of:
linear or nonlinear regression algorithms; linear or nonlinear classification algorithms; ANOVA; neural network algorithms; genetic algorithms; support vector machines algorithms; hierarchical analysis or clustering algorithms; hierarchical algorithms using decision trees; kernel based machine algorithms such as kernel partial least squares algorithms, kernel matching pursuit algorithms, kernel fisher discriminate analysis algorithms, or kernel principal components analysis algorithms; Bayesian probability function algorithms; Markov Blanket algorithms; a plurality of algorithms arranged in a committee network; and forward floating search or backward floating search algorithms.

6. The method according to claim 2 FURTHER **CHARACTERIZED IN THAT**
the correlation to predict the response to endocrine therapy or disease progression is particularly so as to predict the response to tamoxifen or tumor aggressiveness respectively; and
breast cancer in a patient is diagnosed by taking a biopsy of breast cancer tissue and identifying that said biopsy is wholly or partially malignant, identifying clinicopathological values associated with said malignant biopsy, analyzing said malignant tissue for the proteomic markers ER, TP-53, EEBR2, BCL-2, and one or more additional proteomic markers, evaluating the patient's prediction of response of said tumor to said therapy or evaluated risk of disease progression, respectively from said measured levels of proteomic markers and clinicopathological values, and administering tamoxifen, or other therapy as appropriate to the evaluated prediction of response of said tumor to said therapy or evaluated risk of disease progression, respectively.

7. The method according to claim 6 FURTHER **CHARACTERIZED IN THAT** the analysis one or more additional proteomic markers analyzed includes, in addition to markers ER, TP-53, EEBR2, and BCL-2, a proteomic marker of endocrine co-regulation.

8. The method of claim 1 FURTHER **CHARACTERIZED IN THAT**
the analyzing of one or more additional markers of breast cancer disease processes includes, in addition to one or more molecular markers of hormone receptor status, one or more growth factor receptor markers, and one or more tumor suppression molecular markers is of one or more markers selected from the group consisting of two or more of the following: ESR1, PGR, ACTC, AIB1, ANGPT1, AURKA, AURKB, BCL-2, CAV1, CCND1, CCNE, CD44, CDH1, CDH3, CDKN1B, COX2, CTNNA1, CTNNB1, CTSD, EGFR, ERBB2, ERBB2-ALT, ERBB3, ERBB4, EGFR, FGF2, FGFR1, FHIT, GATA3, GATA4, KRT14, KRT5/6, KRT8/18, KRT17, KRT19, MET, MKI67, MLLT4, MME, MMP9, MSN, MTA1, MUC1, MYC, NME1, NRG1, PARK2, PLAU, P-27, S100, SCRIB, TACC1, TACC2, TACC3, THBS1, TIMP1, TP-53, VEGF, VIM or markers related thereto;
the correlation is further so as to determine breast cancer treatment response or prognostic outcome;
the correlation is performed in accordance with an algorithm drawn from the group consisting essentially of: linear or nonlinear regression algorithms; linear or nonlinear classification algorithms; ANOVA; neural network algorithms; genetic algorithms; support vector machines algorithms; hierarchical analysis or clustering algorithms; hierarchical algorithms using decision trees; kernel based machine algorithms such as kernel partial least squares algorithms, kernel matching pursuit algorithms, kernel fisher discriminate analysis algorithms, or kernel principal components analysis algorithms; Bayesian probability function algorithms; Markov Blanket algorithms; recursive feature elimination or entropy-based recursive feature elimination algorithms; a plurality of algorithms arranged in a committee network; and forward floating search or backward floating search algorithms; and
the correlation is to further determine treatment outcome is, in addition to prediction of response to endocrine therapy, expanded so as to predict a response to chemotherapy.

9. A pair of molecular markers, each of which has two conditions, suitably assessed in combination to predict the outcome in endocrine therapy for breast cancer, the molecular marker pair **CHARACTERIZED IN** CONSISTING ESSENTIALLY OF
TP-53, having both a low condition defined as a percentage of positively staining cells <70% and a high condition defined as a percentage of positively staining cells >=70%; and
BCL2 having both a high condition with a score =3, and a low condition with a score of 1 or 2.

10. The molecular marker pair of claim 9 FURTHER **CHARACTERIZED IN** CONSISTING ESSENTIALLY OF
ER, having both a minus condition ER- defined as absence and a positive condition ER+ defined as presence; and
ERBB2, having both a minus condition ERBB2- defined as absence and a positive condition ERBB2+ defined as presence.

11. The molecular marker pair of claim 9 FURTHER **CHARACTERIZED IN** CONSISTING ESSENTIALLY OF
ER, having both a minus condition ER- defined as absence and a positive condition ER+ defined as presence; and
ERBB2, having both a minus condition ERBB2- defined as absence and a positive condition ERBB2+ defined as presence;
wherein the four combinations of (1) ER+ and ERBB2+, (2) ER+ and ERBB2-, (3) ER- and ERBB2+, and (4) ER- and ERBB2-, each predict a different percentage disease specific survival.

12. A kit **CHARACTERIZED IN THAT** are present
a panel of antibodies whose binding with breast cancer tumor samples has been correlated with breast cancer treatment outcome or patient prognosis;
reagents to assist antibodies of said panel of antibodies in binding to tumor samples; and
a computer algorithm, residing on a computer, operating, in consideration of all antibodies of the panel historically analyzed to bind to tumor samples, to interpolate, from the aggregation of all specific antibodies of the panel found bound to the breast cancer tumor sample, a prediction of treatment outcome for a specific treatment for breast cancer or a future risk of breast cancer progression for the subject.

13. The kit according to claim 12 FURTHER **CHARACTERIZED IN THAT** the antibodies include
antibodies specific to ER, PGR, ERBB2, TP-53, BCL-2, KI-67 and MYC.

14. The kit according to claim12 FURTHER **CHARACTERIZED IN THAT** the antibodies of the panel of antibodies include
antibodies correlated with breast cancer progression; and
wherein the computer algorithm includes:
an algorithm using kernel partial least squares.

15. The kit according to claim 12 FURTHER **CHARACTERIZED IN THAT** the antibodies of the panel of antibodies include
antibodies specific to ER, ERBB2, TP-53, BCL-2, KI-67 and p-27.

16. The kit according to claim 12 FURTHER **CHARACTERIZED IN THAT** the antibodies of the panel of antibodies include
antibodies specific to ER, PGR, BCL-2 and ERBB2; with one or more additional markers selected from the group consisting of TP-53, KI-67, and KRT5/6; and with one or more additional markers selected from the group consisting of TP-53, KI-67, and KRT5/6; and with one or more additional markers selected from the group consisting of MSN, C-MYC, CAV1, CTNNB1, CDH1, MME, AURKA, P-27, GATA3, HER4, VEGF, CTNNA1, and CCNE.
